# EUROPEAN PATENT APPLICATION

(11) **EP 3 522 191 A1**
(43) Date of publication of application: **07.08.2019**
(21) Application number: 17855937.3
(22) Date of filing: 21.09.2017
(51) Int. Cl.: H01G 9/20, C09B 57/10, C09B 67/44, C07D 213/38, C07D 213/79, C07F 15/00

(54) **PHOTOELECTRIC CONVERSION ELEMENT, DYE-SENSITIZED SOLAR CELL, METAL COMPLEX DYE, DYE SOLUTION, AND OXIDE SEMICONDUCTOR ELECTRODE**

(30) Priority: 29.09.2016 JP 2016190623
(71) Applicant: FUJIFILM Corporation, Tokyo 106-8620 (JP)
(72) Inventor: SASAKI, Kouitsu, Ashigarakami-gun Kanagawa 258-8577 (JP); WATANABE, Kousuke, Ashigarakami-gun Kanagawa 258-8577 (JP); HAMADA, Kazuhiro, Ashigarakami-gun Kanagawa 258-8577 (JP)
(74) Representative: Hoffmann Eitle
(86) International application number: PCT/JP2017/034115
(87) International publication number: WO 2018/061983

(57) **Abstract**

Provided are a photoelectric conversion element including an electrically conductive support, a photoconductor layer including an electrolyte, a charge transfer layer including an electrolyte, and a counter electrode. The photoconductor layer has semiconductor fine particles having a metal complex dye represented by specific Formula (1) supported thereon; a dye-sensitized solar cell; a metal complex dye; a dye solution; and an oxide semiconductor electrode.

## Description

### BACKGROUND OF THE INVENTION

### 1. Field of the Invention

The present invention relates to a photoelectric conversion element, a dye-sensitized solar cell, a metal complex dye, a dye solution, and an oxide semiconductor electrode.

### 2. Description of the Background Art

Photoelectric conversion elements are used in various photosensors, copying machines, photo electrochemical cells such as solar cells, and the like. These photoelectric conversion elements have adopted various systems to be put into use, such as systems utilizing metals, systems utilizing semiconductors, systems utilizing organic pigments or dyes, or combinations of these elements. In particular, solar cells utilizing inexhaustible solar energy do not necessitate fuels, and full-fledged practicalization of the solar cells using an inexhaustible clean energy is being highly expected. Above all, research and development of silicon-based solar cells have long been in progress, and many countries also support with policy-wise considerations, and thus dissemination of silicon-based solar cells is still in progress. However, silicon is an inorganic material, and thus, naturally has limitations in terms of improvement of throughput, cost, and the like.

Thus, research is being vigorously carried out on photo electrochemical cells (also referred to as dye-sensitized solar cells) using metal complex dyes. In particular, what have built momentum toward such research were the research results from Graetzel et al. of École Polytechnique Fédérale de Lausanne in Switzerland. They have employed a structure in which a dye formed from a ruthenium complex is fixed on the surface of a porous titanium oxide film, and have realized a photoelectric conversion efficiency which is comparable to that of amorphous silicon. Thus, dye-sensitized solar cells that can be produced even without use of expensive vacuum devices have instantly attracted the attention of researchers all over the world.

Hitherto, dyes called N3, N719, and N749 (also referred to as Black Dye), Z907, and J2, and the like have generally been developed as a metal complex dye for use in a dye-sensitized solar cell.

In addition to these metal complex dyes, various metal complex dyes have been studied.

Examples of such other metal complex dyes include a metal complex dye having a bipyridine ligand formed by the bonding of two pyridine rings having a di(4-substituted phenyl)aminostyryl skeleton.

Specifically, for example, JP2001-291534A describes a dye (D-3) having a bipyridine ligand in which each of the two pyridine rings has a 4-diphenylaminostyryl skeleton. Further, JP2008-021496A describes a dye (40) having, as an auxiliary ligand, a bipyridine ligand in which each of the pyridine rings has an N,N'-bis(4-methyl)phenyl-4-aminostyryl group, a 2,2'-bipyridine-4,4'-dicarboxylic acid ligand, and two isothiocyanate groups. In addition, JP2013-072079A describes a dye (D-1-7a) having a bipyridine ligand in which each of the pyridine rings has a diphenylaminostyryl skeleton having a carbon atom at the 4-position of the phenyl group being substituted with a tertiary butyl group.

In addition, J. Mater. Chem., 2009, 19, p. 5364-5376 describes a dye (Ru-TPA-EO-NCS) having a bipyridine ligand in which each of pyridine rings has a styryl skeleton with an asymmetric amino group having a phenyl group substituted with an alkoxy group at a carbon atom at the 4-position and an unsubstituted phenyl group being introduced into a phenyl group.

### SUMMARY OF THE INVENTION

However, performance required for a photoelectric conversion element and a dye-sensitized solar cell has increased, and a further improvement of photoelectric conversion efficiency has been desired. The photoelectric conversion efficiency of the photoelectric conversion element and the dye-sensitized solar cell is determined by production of an open-circuit voltage V_{OC}, a short-circuit current density J_{SC}, and a curve factor (fill factor) FF. Accordingly, in a case where any one of the open-circuit voltage, the short-circuit current density, or the curve factor can be enhanced, it is possible to expect an improvement in the photoelectric conversion efficiency.

The present invention has an object to provide a photoelectric conversion element and a dye-sensitized solar cell, each of which exhibits a high open-circuit voltage, and a metal complex dye, a dye solution, and an oxide semiconductor electrode, each for use in the photoelectric conversion element and the dye-sensitized solar cell.

The present inventors have discovered that in a case where a metal complex dye having a bipyridine ligand having an aminostyryl group with a specific structure at the 4-position of each of pyridine rings, a bipyridine ligand having a carboxyl group or a salt thereof at the 4-position of each of pyridine rings, and two monodentate ligands is used as a sensitizing dye in a photoelectric conversion element and a dye-sensitized solar cell, a high open-circuit voltage is exhibited, as compared with a case where a metal complex dye in the related art is used. Based on this finding, the present inventors have repeated the investigations, leading to completion of the present invention.

That is, the objects of the present invention have been accomplished by the following means.
<1> A photoelectric conversion element comprising:
   an electrically conductive support;
   a photoconductor layer including an electrolyte;
   a charge transfer layer including an electrolyte; and
   a counter electrode,
   in which the photoconductor layer has semiconductor fine particles having a metal complex dye represented by Formula (1) supported thereon.

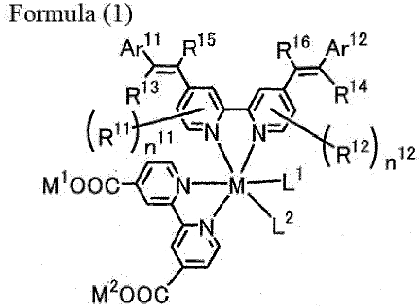

   In the formula, M represents a metal ion.
   R¹¹ and R¹² each independently represent an alkyl group, an alkoxy group, an aryl group, an alkylthio group, a heteroaryl group, an amino group, or a halogen atom. n¹¹ and n¹² each independently represent an integer of 0 to 3.
   R¹³ to R¹⁶ each independently represent a hydrogen atom, an alkyl group, an acyl group, an aryl group, or a heteroaryl group.
   Ar¹¹ and Ar¹² each independently represent a group represented by any one of Formula (2-1) or Formula (2-2), provided that in a case where all of R¹³ to R¹⁶ are each a hydrogen atom or methyl, at least one of Ar¹¹ or Ar¹² represents a group represented by Formula (2-2).
   M¹ and M² each independently represent any one of a proton, a metal cation, or a non-metal cation.
   L¹ and L² each independently represent a monodentate ligand.

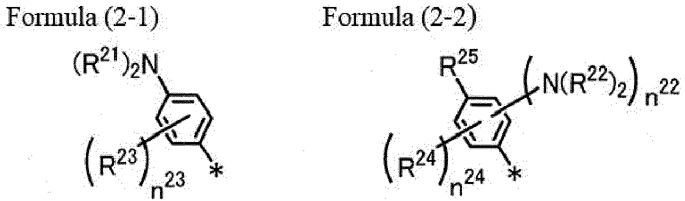

   In the formulae, R²¹ and R²² each independently represent an alkyl group, an aryl group, or a heteroaryl group.
   R²³ represents an alkyl group, an alkoxy group, an aryl group, an alkylthio group, a heteroaryl group, an amino group, or a halogen atom.
   R²⁴ represents an alkyl group, an alkoxy group, an aryl group, an alkylthio group, a heteroaryl group, or a halogen atom.
   R²⁵ represents a hydrogen atom, an alkyl group, an alkoxy group, an aryl group, an alkylthio group, a heteroaryl group, or a halogen atom.
   n²² is an integer of 1 to 4, n²³ is an integer of 0 to 4, and n²⁴ is an integer of 0 to 3. A sum of n²² and n²⁴ is an integer of 1 to 4.
   * represents a bonding moiety to a carbon atom to which R¹³ or R¹⁴ is bonded.
<2> The photoelectric conversion element as described in <1>,
   in which at least one of Ar¹¹ or Ar¹² represents the group represented by Formula (2-2).
<3> The photoelectric conversion element as described in <1> or <2>,
   in which both of Ar¹¹ and Ar¹² each represent the group represented by Formula (2-2).
<4> The photoelectric conversion element as described in any one of <1> to <3>,
   in which R²¹ and R²² each independently represent an alkyl group or an aryl group.
<5> The photoelectric conversion element as described in any one of <1> to <4>,
   in which R²¹ and R²² each independently represent a phenyl group.
<6> The photoelectric conversion element as described in any one of <1> to <5>,
   in which R²¹ and R²² have at least one selected from the group consisting of an alkyl group, an alkoxy group, an aryl group, an alkylthio group, a heteroaryl group, an amino group, and a halogen atom as a substituent.
<7> The photoelectric conversion element as described in any one of <1> to <6>,
   in which R²¹ and R²² are each independently represented by any one of Formula (R2-1), ..., or Formula (R2-5). 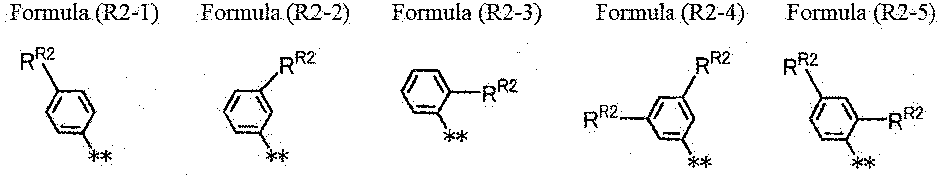
   In the formula, R^{R2} represents an alkyl group, an alkoxy group, an aryl group, an alkylthio group, a heteroaryl group, an amino group, or a halogen atom. ** represents a bonding moiety to N in Formula (2-1) or Formula (2-2).
<8> The photoelectric conversion element as described in any one of <1> to <7>,
   in which all of R¹³ to R¹⁶ are each a hydrogen atom.
<9> The photoelectric conversion element as described in any one of <1> to <7>,
   in which at least one of R¹³, ..., or R¹⁶ represents an alkyl group, an acyl group, an aryl group, or a heteroaryl group.
<10> The photoelectric conversion element as described in <9>,
   in which at least one of a set of R¹³ and R¹⁴ or a set of R¹⁵ and R¹⁶ represents an alkyl group, an acyl group, an aryl group, or a heteroaryl group.
<11> A dye-sensitized solar cell comprising the photoelectric conversion element as described in any one of <1> to <10>.
<12> A metal complex dye represented by Formula (1). 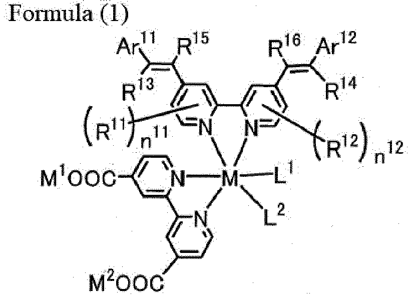
   In the formula, M represents a metal ion.
   R¹¹ and R¹² each independently represent an alkyl group, an alkoxy group, an aryl group, an alkylthio group, a heteroaryl group, an amino group, or a halogen atom. n¹¹ and n¹² each independently represent an integer of 0 to 3.
   R¹³ to R¹⁶ each independently represent a hydrogen atom, an alkyl group, an acyl group, an aryl group, or a heteroaryl group.
   Ar¹¹ and Ar¹² each independently represent a group represented by any one of Formula (2-1) or Formula (2-2), provided that in a case where all of R¹³ to R¹⁶ are each a hydrogen atom or methyl, at least one of Ar¹¹ or Ar¹² represents a group represented by Formula (2-2).
   M¹ and M² each independently represent any one of a proton, a metal cation, or a non-metal cation.
   L¹ and L² each independently represent a monodentate ligand.

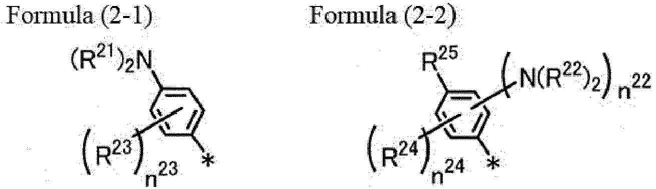

   In the formulae, R²¹ and R²² each independently represent an alkyl group, an aryl group, or a heteroaryl group.
   R²³ represents an alkyl group, an alkoxy group, an aryl group, an alkylthio group, a heteroaryl group, an amino group, or a halogen atom.
   R²⁴ represents an alkyl group, an alkoxy group, an aryl group, an alkylthio group, a heteroaryl group, or a halogen atom.
   R²⁵ represents a hydrogen atom, an alkyl group, an alkoxy group, an aryl group, an alkylthio group, a heteroaryl group, or a halogen atom.
   n²² is an integer of 1 to 4, n²³ is an integer of 0 to 4, and n²⁴ is an integer of 0 to 3. A sum of n²² and n²⁴ is an integer of 1 to 4.
   * represents a bonding moiety to a carbon atom to which R¹³ or R¹⁴ is bonded.
<13> A dye solution comprising:
   the metal complex dye as described in <12>; and
   a solvent.
<14> An oxide semiconductor electrode comprising the metal complex dye as described in <12>.

In the present specification, in a case where a double bond exists in an E configuration or a Z configuration in the molecule, it may be either one of the two configurations or a mixture thereof unless otherwise specified.

In a case where there are a plurality of substituents, linking groups, ligands, or the like (hereinafter referred to as substituents or the like) represented by specific symbols, or in a case where a plurality of substituents and the like are defined at the same time, the respective substituents or the like may be the same as or different from each another unless otherwise specified. This shall also apply to the definition of the number of substituents or the like. Further, in a case where a plurality of substituents and the like are close to each other (in particular, adjacent to each other), they may be linked to each other to form a ring, unless otherwise specified. In addition, a ring, for example, an alicycle, an aromatic ring, or a heterocycle may further be fused to form a fused ring, unless otherwise specified.

In the present specification, reference to a compound (including a complex and a dye) is used in a meaning that encompasses, in addition to the compound itself, salts and ions of the compound. Further, the reference is used to encompass modifications of some of the structure within a scope not interfering with the effects of the present invention. In addition, a compound for which substitution or non-substitution is not explicitly described is meant to indicate that the compound may have an arbitrary substituent within a scope not interfering with the effects of the present invention. This shall also apply to substituents, linking groups, and ligands.

In addition, in the present specification, a numerical value range represented by "(a value) to (a value)" means a range including the numerical values represented before and after "to" as a lower limit value and an upper limit value, respectively.

According to the present invention, it is possible to provide a photoelectric conversion element and a dye-sensitized solar cell, each of which exhibits a high open-circuit voltage. According to the present invention, it is also possible to provide a metal complex dye, a dye solution, and an oxide semiconductor electrode, each of which is suitably used in the photoelectric conversion element and the dye-sensitized solar cell, each of which exhibits the above-mentioned excellent characteristics.

The above or other characteristics and advantages of the present invention will be further clarified from the following description appropriately with reference to drawings attached hereto.

### BRIEF DESCRIPTION OF THE DRAWINGS

Fig. 1 is a cross-sectional view schematically showing a photoelectric conversion element in a first aspect of the present invention, including an enlarged view of the circled portion in a layer thereof, in a system in which the photoelectric conversion element is applied in cell uses.
Fig. 2 is a cross-sectional view schematically showing a dye-sensitized solar cell including a photoelectric conversion element in a second aspect of the present invention.

### DESCRIPTION OF THE PREFERRED EMBODIMENTS

### [Photoelectric Conversion Element and Dye-Sensitized Solar Cell]

The photoelectric conversion element of an embodiment of the present invention has an electrically conductive support, a photoconductor layer including an electrolyte, a charge transfer layer including an electrolyte, and a counter electrode (opposite electrode). The photoconductor layer, the charge transfer layer, and the counter electrode are provided in this order on the electrically conductive support.

In the photoelectric conversion element of the embodiment of the present invention, the semiconductor fine particles forming the photoconductor layer have a metal complex dye represented by Formula (1) which will be described later supported thereon as a sensitizing dye. Here, the aspect in which the metal complex dye is supported on the surface of the semiconductor fine particles encompasses an aspect in which the metal complex dye is deposited on the surface of the semiconductor fine particles, an aspect in which the metal complex dye is adsorbed onto the surface of the semiconductor fine particles, and a mixture of these aspects. The adsorption encompasses chemical adsorption and physical adsorption, with the chemical adsorption being preferable.

Moreover, the photoconductor layer includes an electrolyte. The electrolyte included in the photoconductor layer has the same definition as an electrolyte included in the charge transfer layer which will be described later, and preferred examples thereof are the same. The electrolyte included in the photoconductor layer may be the same as or different from the electrolyte included in the charge transfer layer, but they are preferably the same as each other.

The photoelectric conversion element of the embodiment of the present invention is not particularly limited in terms of configurations other than the configuration defined in the present invention, and may adopt and use known configurations regarding photoelectric conversion elements. The respective layers constituting the photoelectric conversion element of the embodiment of the present invention are designed depending on purposes, and may be formed into, for example, a single layer or multiple layers. Further, layers other than the respective layers may be included, as necessary.

The dye-sensitized solar cell of an embodiment of the present invention is formed of the photoelectric conversion element of the embodiment of the present invention.

Hereinafter, preferred embodiments of the photoelectric conversion element and the dye-sensitized solar cell of the embodiments of the present invention will be described.

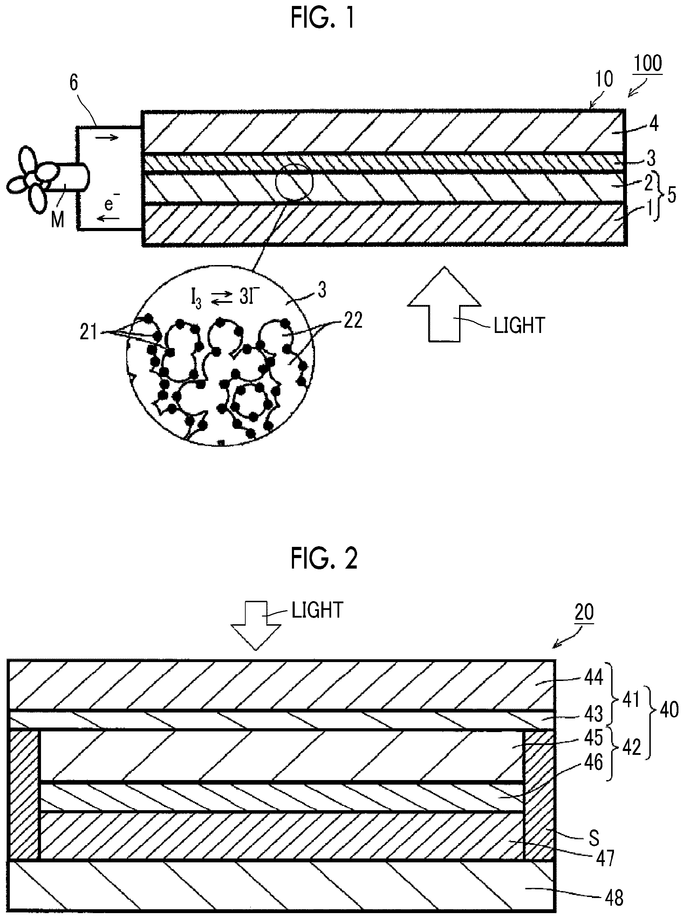

A system 100 shown in Fig. 1 is a system in which a photoelectric conversion element 10 in the first aspect of the present invention is applied in cell uses where an operating means M (for example, an electric motor) in an external circuit 6 is forced to work.

The photoelectric conversion element 10 includes an electrically conductive support 1, a photoconductor layer 2 including semiconductor fine particles 22 sensitized by having a dye (metal complex dye) 21 supported thereon and an electrolyte between the semiconductor fine particles 22, a charge transfer layer 3 that is a hole transport layer, and a counter electrode 4.

In the photoelectric conversion element 10, the photoconductor layer 2 has the metal complex dye represented by Formula (1) adsorbed on the semiconductor fine particles 22, which is also referred to as an oxide semiconductor electrode. Further, the light-receiving electrode 5 has the electrically conductive support 1 and the photoconductor layer 2, and functions as a working electrode.

In the system 100 in which the photoelectric conversion element 10 is applied, light incident to the photoconductor layer 2 excites the metal complex dye 21. The excited metal complex dye 21 has electrons having high energy, and these electrons are transferred from the metal complex dye 21 to a conduction band of the semiconductor fine particles 22, and further reach the electrically conductive support 1 by diffusion. At this time, the metal complex dye 21 is in an oxidant (cation). While the electrons reaching the electrically conductive support 1 work in an external circuit 6, they reach the oxidant of the metal complex dye 21 through the counter electrode 4 and the charge transfer layer 3, and the oxidant is reduced. By repeating a cycle of the excitation of the metal complex dye and the electron movement, the system 100 functions as a solar cell.

A dye-sensitized solar cell 20 shown in Fig. 2 is constituted with the photoelectric conversion element in the second aspect of the present invention.

The photoelectric conversion element which serves as the dye-sensitized solar cell 20 is different from the photoelectric conversion element shown in Fig. 1 in terms of the configurations of the electrically conductive support 41 and the photoconductor layer 42, and incorporation of a spacer S, but except for these points, has the same configuration as the photoelectric conversion element 10 shown in Fig. 1. That is, the electrically conductive support 41 has a bilayered structure including a substrate 44 and a transparent electrically-conductive film 43 which is formed on the surface of the substrate 44. Further, the photoconductor layer 42 has a bilayered structure including a semiconductor layer 45 and a light-scattering layer 46 which is formed adjacent to the semiconductor layer 45. The photoconductor layer 42 has at least the metal complex dye represented by Formula (1), adsorbed on semiconductor fine particles which form the photoconductor layer 42, and is also referred to as an oxide semiconductor electrode. A spacer S is provided between the electrically conductive support 41 and the counter electrode 48. In the dye-sensitized solar cell 20, 40 is a light-receiving electrode and 47 is a charge transfer layer.

In a similar manner to the system 100 in which the photoelectric conversion element 10 is applied, the dye-sensitized solar cell 20 functions as a solar cell by light incident on the photoconductor layer 42.

The photoelectric conversion element and the dye-sensitized solar cell of the embodiments of the present invention exhibit excellent photoelectric conversion efficiency even in a low-illumination environment. Accordingly, they are suitably used even in a low-illumination environment. In this case, a dye-sensitized solar cell using the photoelectric conversion element of the embodiment of the present invention is also referred to as a dye-sensitized photoelectrochemical cell.

A low-illumination environment is an environment with a lower illuminance than that of solar light in clear weather (an environment with an illuminance of 10,000 lux or less), and examples thereof include a low-illumination solar light environment in cloudy weather, rainy weather, or the like, an indoor environment, and an environment with an illumination device such as a fluorescent light lamp.

The photoelectric conversion element and the dye-sensitized solar cell of the embodiments of the present invention are not limited to the above-mentioned preferred embodiment, and the configuration and the like of the respective embodiments can be appropriately combined among the respective aspects while departing from the scope of the present invention.

In the present invention, the materials and the respective members for use in the photoelectric conversion element and the dye-sensitized solar cell can be prepared by ordinary methods. Reference can be made to, for example, US4927721A, US4684537A, US5084365A, US5350644A, US5463057A, US5525440A, JP1985-249790A (JP-H07-249790A), JP2001-185244A, JP2001-210390A, JP2003-217688A, JP2004-220974A, and JP2008-135197A.

### <Metal Complex Dyes Represented by Formula (1)>

The metal complex dye of the embodiment of the present invention is represented by Formula (1). In a case where the metal complex dye of the embodiment of the present invention is used in a photoelectric conversion element and a dye-sensitized solar cell, a high open-circuit voltage can be provided. Therefore, the metal complex dye of the embodiment of the present invention is preferably used as sensitizing dye in a dye-sensitized solar cell.

In the present invention, in a case where the metal complex dye represented by Formula (1) exists as an isomer such as an optical isomer, a geometric isomer, a linkage isomer, and an ionized isomer, it may be either any of these isomers or a mixture of these isomers.

Details of a reason why the metal complex dye represented by Formula (1) can provide the photoelectric conversion element and the dye-sensitized solar cell with the excellent performance are still not clear, but are considered to be as follows.

In the metal complex dye represented by Formula (1), at least one of Ar¹¹ or Ar¹² has the group represented by Formula (2-2) which will be described later, or R¹³ to R¹⁶ have the substituent which will be described later. In a case where the metal complex dye of the embodiment of the present invention having such a chemical structure is supported on the semiconductor fine particles, an oxidant (for example, I₃⁻ in a case where the redox couple is formed of a combination of iodine and iodide) of a redox couple included in the charge transfer layer can be prevented from penetrating into the metal complex dye and being close to or in contact with the semiconductor fine particles. It is considered that by the effect of preventing the intrusion of the oxidant, a reverse electron transfer to the oxidant of the redox couple formed of the semiconductor fine particles is suppressed, and thus, a high open-circuit voltage can be exerted to the photoelectric conversion element or the dye-sensitized solar cell.

Furthermore, it is considered that the metal complex dye represented by Formula (1) is less likely to aggregate, prevents inefficient processes (an electron trap or the like in the semiconductor fine particles), and thus contributes to a further improvement of the open-circuit voltage. It is presumed that suppression of the aggregation of the dye is due to reduction in flatness of conjugated chains caused by steric hindrance occurring in a case where R¹³ to R¹⁶ are each a substituent, or ease of a change in three-dimensional structures by the rotation of a single bond between Ar¹¹ and an sp² carbon atom in a case where Ar¹¹ or the like has the group represented by Formula (2-2).

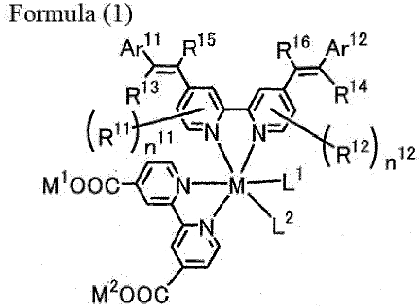

In Formula (1), M represents a metal ion, and examples thereof include ions of each of the elements belonging to Groups 6 to 12 on the long-form periodic table of the elements. Examples of such metal ions include respective ions of Ru, Fe, Os, Cu, W, Cr, Mo, Ni, Pd, Pt, Co, Ir, Rh, Re, Mn, and Zn. The metal ion M may be one kind of ion, or two or more kinds of ions.

In the present invention, the metal ion M is preferably Os²⁺, Ru²⁺, or Fe²⁺, more preferably Os²⁺ or Ru²⁺, and particularly preferably Ru²⁺ among them.

In addition, in a state where M is incorporated in the photoelectric conversion element, the valence of M may be changed by a redox reaction with the surrounding material in some cases.

R¹¹ and R¹² each represent an alkyl group, an alkoxy group, an aryl group, an alkylthio group, a heteroaryl group, an amino group, or a halogen atom. Among those, the alkyl group, the alkoxy group, the aryl group, or the halogen atom is preferable, and the alkyl group or the aryl group is more preferable.

Examples of each of the groups that can be adopted as R¹¹ and R¹² include the corresponding groups in the substituent group T which will be described later, and preferred examples thereof are the same.

n¹¹ and n¹² are each an integer of 0 to 3, preferably 0 or 1, and more preferably 0.

R¹¹ and R¹² may be the same as or different from each other.

Each of the groups which can be adopted as R¹¹ and R¹² may further have a substituent. The substituent which may further be contained is not particularly limited, but is preferably a substituent selected from the substituent group T which will be described later.

In addition, it is preferable that each of the groups which can be adopted as R¹¹ and R¹² does not have an acidic group which will be described later or a salt thereof.

R¹³ to R¹⁶ each represent a hydrogen atom, an alkyl group, an acyl group, an aryl group, or a heteroaryl group.

Examples of such a group which can be adopted as R¹³ to R¹⁶ each include the corresponding groups in the substituent group T which will be described later, and preferred examples thereof are the same. However, in a case where all of R¹³ to R¹⁶ are each an alkyl group, the number of carbon atoms of the alkyl group is preferably 2 or more, and can also be 3 or more.

Among those, R¹³ to R¹⁶ are each preferably a hydrogen atom, an alkyl group, an aryl group, or a heteroaryl group.

R¹³ to R¹⁶ are each selected from the group consisting of a hydrogen atom, an alkyl group, an acyl group, an aryl group, and a heteroaryl group, but an aspect in which all of R¹³ to R¹⁶ are each a hydrogen atom (referred to as a non-substitution aspect), and an aspect in which at least one of R¹³, ..., or R¹⁶ is selected from the group consisting of an alkyl group, an acyl group, an aryl group, and a heteroaryl group (referred to as a substitution aspect) are preferable.

In the substitution aspect, the number of the substituents selected from the group, which can be adopted as R¹³ to R¹⁶, may be any one of 1 to 4, and is preferably 2 or 4, and more preferably 2. In this case, any one of R¹³ to R¹⁶ may be a substituent selected from the group, but at least one of a set of R¹³ and R¹⁴ or a set of R¹⁵ and R¹⁶ is preferably a substituent selected from the group. The substituent selected from the group is preferably an alkyl group, an aryl group, or a heteroaryl group.

R¹³ to R¹⁶ (substituents selected from the group) may be the same as or different from each other, and are preferably the same as each other.

Each of the groups which can be adopted as R¹³ to R¹⁶ may further have a substituent. The substituent which may further be contained is not particularly limited, but is preferably a substituent selected from the substituent group T which will be described later. For example, an alkyl group (including halogenated alkyl group which is further substituted with a halogen atom), an alkoxy group, an amino group, or a halogen atom is preferable.

In addition, it is preferable that each of the groups which can be adopted as R¹³ to R¹⁶ does not have the acidic group which will be described later or a salt thereof.

In Formula (1), Ar¹¹ and Ar¹² each represent a group represented by any one of Formula (2-1) or Formula (2-2).

However, in a case where all of R¹³ to R¹⁶ are each a hydrogen atom or methyl, at least one of Ar¹¹ or Ar¹² represents a group represented by Formula (2-2). Further, in a case where at least one of R¹³, ..., or R¹⁶ is methyl and the others are each a hydrogen atom, it is preferable that at least one of Ar¹¹ or Ar¹² is a group represented by Formula (2-2).

Irrespective of R¹³ to R¹⁶, at least one of Ar¹¹ or Ar¹² is preferably a group represented by Formula (2-2), and more preferably both of Ar¹¹ and Ar¹² are each the group represented by Formula (2-2).

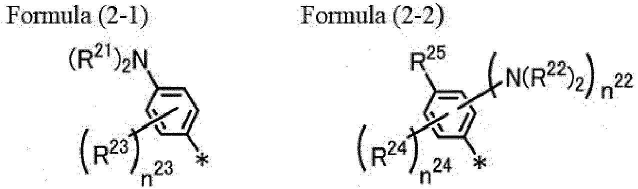

In the formulae, * represents a bonding moiety to a carbon atom to which R¹³ or R¹⁴ is bonded.

R²¹ and R²² each represent an alkyl group, an aryl group, or a heteroaryl group, and are each preferably an alkyl group or an aryl group, more preferably an aryl group, and still more preferably a phenyl group.

Examples of the alkyl group which can be adopted as R²¹ and R²² include a linear alkyl group, a branched alkyl group, and a cyclic (cyclo) alkyl group. The number of carbon atoms of the linear alkyl group or the branched alkyl group is preferably 1 to 30, more preferably 2 to 26, still more preferably 3 to 20, and particularly preferably 3 to 12. The number of carbon atoms of the cyclic alkyl group is preferably 3 to 30, more preferably 5 to 30, still more preferably 6 to 26, and particularly preferably 6 to 20. The cyclic alkyl group may be fused with an alicycle, an aromatic ring, or a heterocycle.

The aryl group which can be adopted as R²¹ and R²² is a group formed of an aromatic hydrocarbon ring, and examples thereof include a monocyclic phenyl group and a fused polycyclic group. The number of carbon atoms of the aryl group is preferably 6 to 30, more preferably 6 to 10, and particularly preferably 6. Examples of the fused polycyclic group include a naphthyl group.

The heteroaryl group which can be adopted as R²¹ and R²² has the same definition as the heteroaryl group in the substituent group T which will be described later, and preferred examples thereof are the same.

Each of the groups which can be adopted as R²¹ and R²² may further have a substituent, and it is preferable that each of the groups which can be adopted as R²² further has a substituent.

The substituent which may further be contained in each of the groups is not particularly limited, and is preferably a substituent selected from the substituent group T which will be described later. Among those, as the substituent, a substituent having at least one selected from the group consisting of an alkyl group, an alkoxy group, an aryl group, an alkylthio group, a heteroaryl group, an amino group, and a halogen atom is more preferable, and a substituent having at least one selected from the group consisting of an alkyl group, an alkoxy group, an aryl group, an alkylthio group, an amino group, and a halogen atom is still more preferable.

The alkyl group and the aryl group which can be adopted as the substituent have the same definitions as the alkyl group and the aryl group which can be adopted as R²¹ and R²², respectively, and preferred examples thereof are the same.

The alkyl moiety of the alkoxy group and the alkylthio group which can be adopted as the substituent has the same definition as the alkyl group which can be adopted as the substituent, and preferred examples thereof are the same.

Examples of the heteroaryl group, the amino group, and the halogen atom which can be adopted as the substituent each include the corresponding group in the substituent group T which will be described later, and preferred examples thereof are the same.

The substituent may further be substituted with the substituent selected from the substituent group T. Examples of the substituent further having a substituent, which can be adopted as R²¹ and R²², include a halogenated alkyl group, and preferably a fluorinated alkyl group.

The number of the substituents contained in each of the groups which can be adopted as R²¹ and R²² is not particularly limited as long as it is 1 or more, and is preferably 1 to 10, more preferably 1 to 5, and still more preferably 1 or 2.

The substitution position in each of the groups which can be adopted as R²¹ and R²² is not particularly limited.

In a case where R²¹ and R²² are each a phenyl group having a substituent, R²¹ and R²² are each preferably represented by any one of Formula (R2-1), ..., or Formula (R2-5), and more preferably represented by Formula (R2-1) or Formula (R2-2).

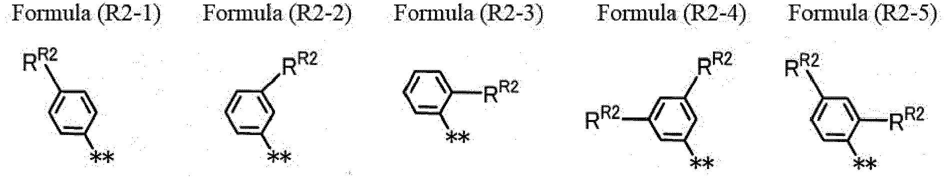

In the formulae, R^{R2} represents an alkyl group, an alkoxy group, an aryl group, an alkylthio group, a heteroaryl group, an amino group, or a halogen atom. R² has the same definition as the substituent which may be contained in each of the groups which can be adopted as R²¹ and R²², and preferred examples thereof are also the same. In Formula (R2-4) and Formula (R2-5), two R^{R2}'s may be the same as or different from each other.
** represents a bonding moiety to N in Formula (2-1) or Formula (2-2).

R²¹ and R²², R²¹ and R²¹, R²² and R²², or R²¹ and R²² may be bonded to each other directly or via a linking group to form a ring. The linking group is not particularly limited, and examples thereof include -O-, -S-, -NR^{NR}-, -C(R^{NR})₂-, and -Si(R^{NR})₂-. Here, R^{NR} represents a hydrogen atom or a group selected from the substituent group T.

In addition, R²¹ and R²² may each be bonded directly or via a linking group to a benzene ring in Formula (2-1) or Formula (2-2) to form a ring.

R²¹ and R²² may be the same as or different from each other, and are preferably the same as each other.

In addition, it is preferable that each of the groups which can be adopted as R²¹ and R²² does not have the acidic group which will be described later or a salt thereof.

n²² is an integer of 1 to 4, and preferably 1 or 2.

n²² may be any integer in the range, but a sum of n²² and n²⁴ which will be described later is an integer of 1 to 4, preferably an integer of 1 to 3, and more preferably 1 or 2.

R²³ represents an alkyl group, an alkoxy group, an aryl group, an alkylthio group, a heteroaryl group, an amino group, or a halogen atom.

R²⁴ represents an alkyl group, an alkoxy group, an aryl group, an alkylthio group, a heteroaryl group, or a halogen atom.

R²³ and R²⁴ are each preferably an alkyl group, an alkoxy group, an aryl group, or a halogen atom, and more preferably an alkyl group or an aryl group.

Examples of each of the groups which can be adopted as R²³ and R²⁴ include the corresponding groups in the substituent group T which will be described later, and preferred examples thereof are the same.

It is preferable that each of the groups which can be adopted as R²³ and R²⁴ does not have the acidic group which will be described later or a salt thereof.

n²³ is an integer of 0 to 4, preferably an integer of 0 to 2, and more preferably 0 or 1.

n²⁴ is an integer of 0 to 3, preferably an integer of 0 to 2, and more preferably 0 or 1.

R²⁵ represents a hydrogen atom, an alkyl group, an alkoxy group, an aryl group, an alkylthio group, a heteroaryl group, or a halogen atom. Among those, the hydrogen atom, the alkyl group, the aryl group, or the halogen atom is preferable, the hydrogen atom or the alkyl group is more preferable, and the hydrogen atom is still more preferable.

Examples of each of the groups which can be adopted as R²⁵ include the corresponding groups in the substituent group T which will be described later, and preferred examples thereof are the same.

It is preferable that each of the groups which can be adopted as R²⁵ does not have the acidic group which will be described later or a salt thereof.

In Formula (1), the pyridine ring having Ar¹¹ and the pyridine ring having Ar¹² may be the same as or different from each other, and are preferably the same as each other.

It is preferable that each of the pyridine ring having Ar¹¹ and the pyridine ring having Ar¹² does not have the acidic group which will be described later or a salt thereof.

In Formula (1), L¹ and L² are not particularly limited as long as they are each a monodentate ligand, and are each preferably, for example, a group or atom selected from the group consisting of an acyloxy group, an acylthio group, a thioacyloxy group, a thioacylthio group, an acylaminooxy group, a thiocarbamate group, a dithiocarbamate group, a thiocarbonate group, a dithiocarbonate group, a trithiocarbonate group, an acyl group, a thiocyanate group, an isothiocyanate group, a cyanate group, an isocyanate group, a cyano group, an alkylthio group, an arylthio group, an alkoxy group, an aryloxy group, and a halogen atom, or anions thereof.

In a case where the ligands L¹ and L² each include an alkyl group, an alkenyl group, an alkynyl group, an alkylene group, or the like, they may be linear or branched, and may or may not have a substituent. Further, in a case where a group capable of adopting a cyclic structure, such as an aryl group, a heterocyclic group, and a cycloalkyl group, is included, these may or may not have a substituent, and may be a monocycle or be fused to form a ring.

Among those, the ligands L¹ and L² are each preferably a cyanate group, an isocyanate group, a thiocyanate group, or an isothiocyanate group, or an anion thereof, more preferably an isocyanate group (isocyanate anion) or an isothiocyanate group (isothiocyanate anion), and particularly preferably an isothiocyanate group (isothiocyanate anion).

L¹ and L² may be the same as or different from each other, and are preferably the same as each other.

M¹ and M² each represent any one of a proton (hydrogen atom), a metal cation, or a non-metal cation. M¹ and M² are each preferably a non-metal cation from the viewpoints of improvement of the photoelectric conversion efficiency, and furthermore, reduction in the unbalance in performance among elements, and preferably a proton or a metal cation from the viewpoint of durability.

The metal cation which can be adopted as M¹ and M² is not particularly limited, but examples thereof include an alkali metal ion, an alkaline earth metal ion, and a metal complex ion. Among these, the alkali metal ion or the alkaline earth metal ion is preferable, the alkali metal ion is more preferable, a lithium ion, a sodium ion, or a potassium ion is still more preferably, and the sodium ion or the potassium ion is particularly preferable.

The non-metal cation which can be adopted as M¹ and M² is not particularly limited, but examples thereof include inorganic or organic ammonium ions (for example, a trialkylammonium ion, a tetraalkylammonium ion, or the like), a phosphonium ion (for example, a tetraalkylphosphonium ion, an alkyltriphenylphosphonium ion, or the like), a pyridinium ion, an imidazolium ion, an amidinium ion, and a guanidinium ion.

Among these, organic ammonium ions (triethylammonium ion, a tetraethylammonium ion, a tetrabutylammonium ion, a tetrahexylammonium ion, a tetraoctylammonium ion, a tetradecylammonium ion, a tetradodecylammonium ion, and the like), a pyridinium ion, an imidazolium ion, or an amidinium ion is preferable, an organic ammonium ion, a pyridinium ion, or an imidazolium ion is more preferable, and organic ammonium ions are still more preferable.

M¹ and M² may be the same as or different from each other.

It is preferable that the metal complex dye represented by Formula (1) does not have an acidic group or a salt thereof, in addition to -COOM¹ and -COOM².

In the present invention, the acidic group is a substituent having a dissociative proton, which has a pKa of 11 or less. The pKa of the acidic group can be determined in accordance with the "SMD/M05-2X/6-31G*" method described in J. Phys. Chem. A2011, 115, p. 6641-6645. Examples of the acidic group include a carboxyl group (-COOH), a phosphonyl group (-PO(OH)₂), a phosphoryl group (-O-PO(OH)₂), a sulfo group (-SO₃H), a boric acid group, a (phenolic) hydroxyl group, a (phenolic) thiol group (mercapto group), and a sulfonamide group.

The salt of the acidic group may be either a metal salt or a non-metal salt. A counter ion in a case where the acidic group is a salt is not particularly limited, but examples thereof include metal cations or non-metal cations which can be adopted as M¹ and M².

The metal complex dye represented by Formula (1) can be synthesized by, for example, the methods described in JP2001-291534A, JP2008-021496A, JP2013-072079A, or J. Mater. Chem., 2009, 19, p. 5364-5376, the patent documents regarding solar cells, known methods, or the methods equivalent thereto.

The metal complex dye represented by Formula (1) has a maximum absorption wavelength in a solution, preferably in a range from 300 to 1,000 nm, more preferably in a range from 350 to 950 nm, and particularly preferably in a range from 370 to 900 nm.

### <Substituent Group T>

In the present invention, preferred examples of the substituent include the groups selected from the following substituent group T.

Incidentally, in the present specification, a case where there is only a simple description of a substituent is intended to refer to this substituent group T, and further, in a case where each of the groups, for example, an alkyl group is merely described, a preferable range and specific examples for the corresponding group for the substituent group T are applied.

Moreover, in the present specification, in a case where an alkyl group is described as different from a cyclic (cyclo)alkyl group, the alkyl group is used to mean inclusion of both of a linear alkyl group and a branched alkyl group. On the other hand, in a case where an alkyl group is not described as different from a cycloalkyl group (a case where an alkyl group is simply described), and unless otherwise specified, the alkyl group is used to mean any of a linear alkyl group, a branched alkyl group, and a cycloalkyl group. This shall apply to a group (an alkoxy group, an alkylthio group, an alkenyloxy group, and the like) including a group (an alkyl group, an alkenyl group, an alkynyl group, and the like) which can adopt a cyclic structure, and a compound including a group which can adopt a cyclic structure. In the following description of the substituent group, for example, a group with a linear or branched structure and a group with a cyclic structure may be sometimes separately described for clarification of both groups, as in the alkyl group and the cycloalkyl group.

Examples of the groups included in the substituent group T include the following groups, or groups formed by combination of a plurality of the following groups:
an alkyl group (preferably an alkyl group having 1 to 20 carbon atoms, and more preferably an alkyl group having 1 to 12 carbon atoms, for example, methyl, ethyl, n-propyl, i-propyl, n-butyl, t-butyl, pentyl, heptyl, 1-ethylpentyl, benzyl, 2-ethoxyethyl, 1-carboxymethyl, or trifluoromethyl), an alkenyl group (preferably an alkenyl group having 2 to 20 carbon atoms, and more preferably an alkenyl group having 2 to 12 carbon atoms, for example, vinyl, allyl, or oleyl), an alkynyl group (preferably an alkynyl group having 2 to 20 carbon atoms, and more preferably an alkynyl group having 2 to 12 carbon atoms, for example, ethynyl, butynyl, or phenylethynyl), a cycloalkyl group (preferably a cycloalkyl group having 3 to 20 carbon atoms), an cycloalkenyl group (preferably a cycloalkenyl group having 5 to 20 carbon atoms), an aryl group (preferably an aryl group having 6 to 26 carbon atoms, for example, phenyl, 1-naphthyl, 4-methoxyphenyl, 2-chlorophenyl, 3-methylphenyl, difluorophenyl, or tetrafluorophenyl), a heterocyclic group (preferably a heterocyclic group having 2 to 20 carbon atoms, more preferably a 5- or 6-membered heterocyclic group having at least one oxygen atom, sulfur atom, or nitrogen atom; and examples of the heterocycle include an aromatic ring and an aliphatic ring. Examples of the aromatic heterocyclic group (a heteroaryl group) include the following groups. For example, 2-pyridyl, 4-pyridyl, 2-imidazolyl, 2-benzimidazolyl, 2-thienyl, 2-furanyl, 2-thiazolyl, or 2-oxazolyl), an alkoxy group (preferably an alkoxy group having 1 to 20 carbon atoms, and more preferably an alkoxy group having 1 to 12 carbon atoms, for example, methoxy, ethoxy, isopropyloxy, or benzyloxy), an alkenyloxy group (preferably an alkenyloxy group having 2 to 20 carbon atoms, and more preferably an alkenyloxy group having 2 to 12 carbon atoms), an alkynyloxy group (preferably an alkynyloxy group having 2 to 20 carbon atoms, and more preferably an alkynyloxy group having 2 to 12 carbon atoms), a cycloalkyloxy group (preferably a cycloalkyloxy group having 3 to 20 carbon atoms), an aryloxy group (preferably an aryloxy group having 6 to 26 carbon atoms), a heterocyclic oxy group (preferably a heterocyclic oxy group having 2 to 20 carbon atoms),
an alkoxycarbonyl group (preferably an alkoxycarbonyl group having 2 to 20 carbon atoms), a cycloalkoxycarbonyl group (preferably a cycloalkoxycarbonyl group having 4 to 20 carbon atoms), an aryloxycarbonyl group (preferably an aryloxycarbonyl group having 6 to 20 carbon atoms), an amino group (preferably an amino group having 0 to 20 carbon atoms, including an alkylamino group, an alkenylamino group, an alkynylamino group, a cycloalkylamino group, a cycloalkenylamino group, an arylamino group, and a heterocyclic amino group, for example, amino, N,N-dimethylamino, N,N-diethylamino, N-ethylamino, N-allylamino, N-(2-propynyl)amino, N-cyclohexylamino, N-cyclohexenylamino, anilino, pyridylamino, imidazolylamino, benzimidazolylamino, thiazolylamino, benzothiazolylamino, or triazinylamino), a sulfamoyl group (preferably a sulfamoyl group having 0 to 20 carbon atoms, preferably an alkyl-, cycloalkyl-, or aryl-sulfamoyl group), an acyl group (preferably an acyl group having 1 to 20 carbon atoms), an acyloxy group (preferably an acyloxy group having 1 to 20 carbon atoms), a carbamoyl group (preferably a carbamoyl group having 1 to 20 carbon atoms, preferably an alkyl-, cycloalkyl-, or aryl-carbamoyl group),
an acylamino group (preferably an acylamino group having 1 to 20 carbon atoms), a sulfonamido group (preferably a sulfonamido group having 0 to 20 carbon atoms, and preferably an alkyl-, cycloalkyl-, or aryl-sulfonamido group), an alkylthio group (preferably an alkylthio group having 1 to 20 carbon atoms, and more preferably an alkylthio group having 1 to 12 carbon atoms, for example, methylthio, ethylthio, isopropylthio, or benzylthio), a cycloalkylthio group (preferably a cycloalkylthio group having 3 to 20 carbon atoms), an arylthio group (preferably an arylthio group having 6 to 26 carbon atoms), an alkyl-, cycloalkyl-, or aryl-sulfonyl group (preferably an alkyl-, cycloalkyl-, or aryl-sulfonyl group having 1 to 20 carbon atoms),
a silyl group (preferably a silyl group having 1 to 20 carbon atoms, preferably an alkyl-, aryl-, alkoxy-, and aryloxy-substituted silyl group), a silyloxy group (preferably a silyloxy group having 1 to 20 carbon atoms, preferably an alkyl-, aryl-, alkoxy-, and aryloxy-substituted silyloxy group), a hydroxyl group, a cyano group, a nitro group, a halogen atom (for example, a fluorine atom, a chlorine atom, a bromine atom, or iodine atom), a carboxyl group, a sulfo group, a phosphonyl group, a phosphoryl group, and a boric acid group.

Examples of the group selected from the substituent group T more preferably include a group other than an acidic group or a salt thereof, still more preferably an alkyl group, an alkenyl group, a cycloalkyl group, an aryl group, a heterocyclic group, an alkoxy group, a cycloalkoxy group, an aryloxy group, an alkoxycarbonyl group, a cycloalkoxycarbonyl group, an amino group, an acylamino group, a cyano group, and a halogen atom; and particularly preferably include an alkyl group, an alkenyl group, a heterocyclic group, an alkoxy group, an alkoxycarbonyl group, an amino group, an acylamino group, and a cyano group.

In a case where the compound, the substituent, or the like includes an alkyl group, an alkenyl group, or the like, these may be substituted or unsubstituted. Further, in a case where the compound, the substituent, or the like includes an aryl group, a heterocyclic group, or the like, these may be a monocycle or a fused ring, and may be substituted or unsubstituted.

Specific examples of the metal complex dye represented by Formula (1) are shown below and in Examples, but the present invention is not limited to these metal complex dyes. In a case where these metal complex dyes are present as optical isomers or geometric isomers, the metal complex dye may be any of these isomers or a mixture of these isomers. In a case where in the following specific examples, any one of M¹ and M² is a metal cation or a non-metal cation, for the sake of convenience, M¹ is described as the metal cation or the non-metal cation. The present invention is not limited to such a case, M² may be a metal cation or a non-metal cation, and a mixture of M¹ being a metal cation or a non-metal cation and M² of a metal cation or a non-metal cation may also be available (the mixing ratio is not particularly limited).

In the following specific examples, * of D¹, D², Ar¹¹, and Ar¹² represents a bonding moiety to a double bond (vinyl group) in Formula (1-1) or Formula (1-2). In addition, * of R¹¹² and R¹²² in Formula (1-3) each represents a bonding moiety to a pyridine ring.

Furthermore, Me represents methyl, Et represents ethyl, nPr represents n-propyl, nBu represents n-butyl, and Ph represents phenyl.

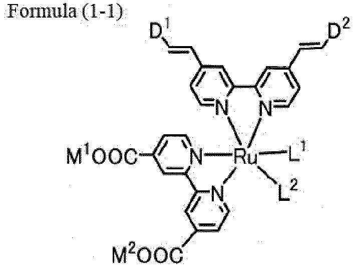

| Metal complex dye | **M¹** | **M²** | **L¹** | **L²** | **D¹** | **D²** |
|---|---|---|---|---|---|---|
| **D-1** | **H** | **H** | **-NCS** | **-NCS** | 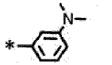 | 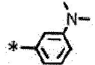 |
| **D-2** | **H** | **H** | **-NCS** | **-NCS** | 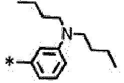 | 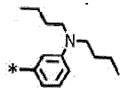 |
| **D-3** | **H** | **H** | **-NCS** | **-NCS** | 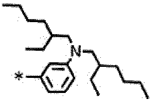 | 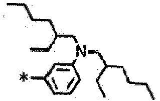 |
| **D-4** | **H** | **H** | **-NCS** | **-NCS** | 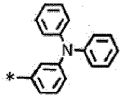 | 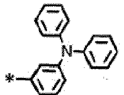 |
| **D-5** | **H** | **H** | **-NCS** | **-NCS** | 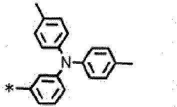 | 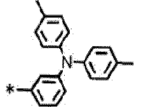 |
| **D-6** | **H** | **H** | **-NCS** | **-NCS** | 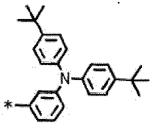 | 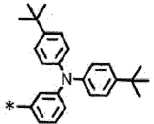 |
| **D-7** | **H** | **H** | **-NCS** | **-NCS** | 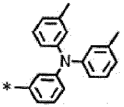 | 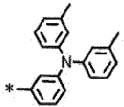 |
| **D-8** | **H** | **H** | **-NCS** | **-NCS** | 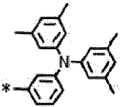 | 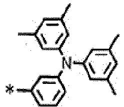 |
| **D-9** | **H** | **H** | **-NCS** | **-NCS** | 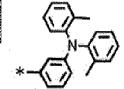 | 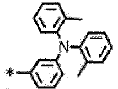 |
| **D-10** | **H** | **H** | **-NCS** | **-NCS** | 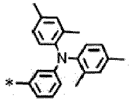 | 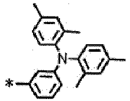 |
| **D-11** | **H** | **H** | **-NCS** | **-NCS** | 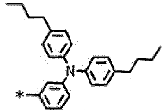 | 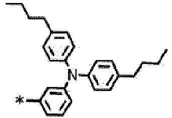 |
| **D-12** | **H** | **H** | **-NCS** | **-NCS** | 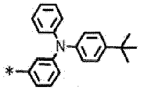 | 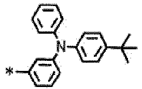 |
| **D-13** | **H** | **H** | **-NCS** | **-NCS** | 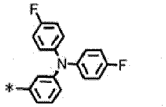 | 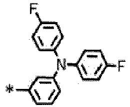 |
| **D-14** | **H** | **H** | **-NCS** | **-NCS** | 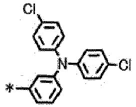 | 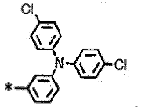 |
| **D-15** | **H** | **H** | **-NCS** | **-NCS** | 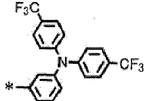 | 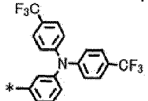 |
| **D-16** | **H** | **H** | **-NCS** | **-NCS** | 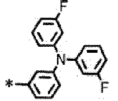 | 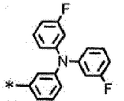 |
| **D-17** | **H** | **H** | **-NCS** | **-NCS** | 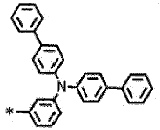 | 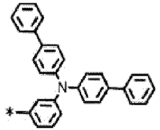 |
| **D-18** | **H** | **H** | **-NCS** | **-NCS** | 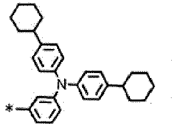 | 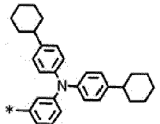 |
| **D-19** | **H** | **H** | **-NCS** | **-NCS** | 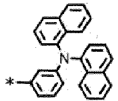 | 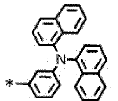 |
| **D-20** | **H** | **H** | **-NCS** | **-NCS** | 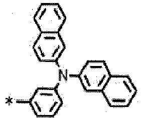 | 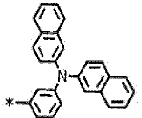 |
| **D-21** | **H** | **H** | **-NCS** | **-NCS** | 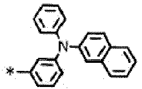 | 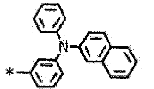 |
| **D-22** | **H** | **H** | **-NCS** | **-NCS** | 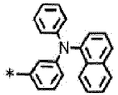 | 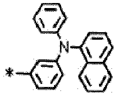 |
| **D-23** | **H** | **H** | **-NCS** | **-NCS** | 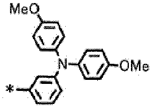 | 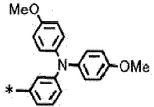 |
| **D-24** | **H** | **H** | **-NCS** | **-NCS** | 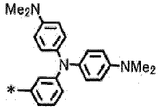 | 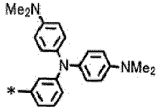 |
| **D-25** | **H** | **H** | **-NCS** | **-NCS** | 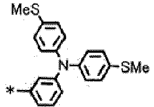 | 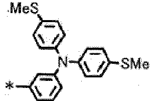 |
| **D-26** | **H** | **H** | **-NCS** | **-NCS** | 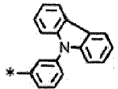 | 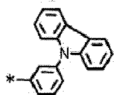 |
| **D-27** | **H** | **H** | **-NCS** | **-NCS** | 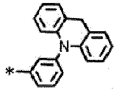 | 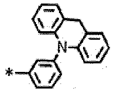 |
| **D-28** | **H** | **H** | **-NCS** | **-NCS** | 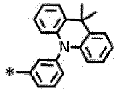 | 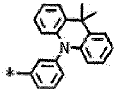 |
| **D-29** | **H** | **H** | **-NCS** | **-NCS** | 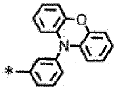 | 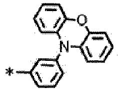 |
| **D-30** | **H** | **H** | **-NCS** | **-NCS** | 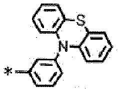 | 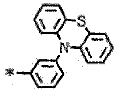 |
| **D-31** | **H** | **H** | **-NCS** | **-NCS** | 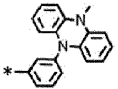 | 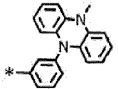 |
| **D-32** | **H** | **H** | **-NCS** | **-NCS** | 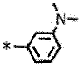 | 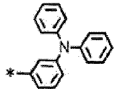 |
| **D-33** | **H** | **H** | **-NCS** | **-NCS** | 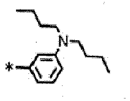 | 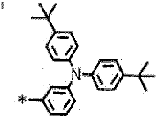 |
| **D-34** | **H** | **H** | **-NCS** | **-NCS** | 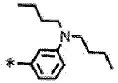 | 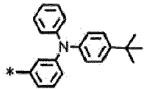 |
| **D-35** | **H** | **H** | **-NCS** | **-NCS** | 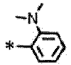 | 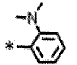 |
| **D-36** | **H** | **H** | **-NCS** | **-NCS** | 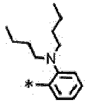 | 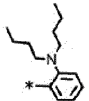 |
| **D-37** | **H** | **H** | **-NCS** | **-NCS** | 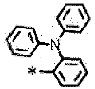 | 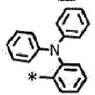 |
| **D-38** | **H** | **H** | **-NCS** | **-NCS** | 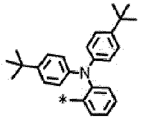 | 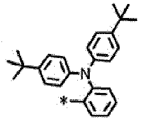 |
| **D-39** | **H** | **H** | **-NCS** | **-NCS** | 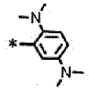 | 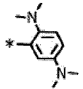 |
| **D-40** | **H** | **H** | **-NCS** | **-NCS** | 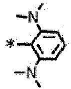 | 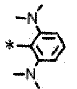 |
| **D-41** | **H** | **H** | **-NCS** | **-NCS** | 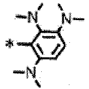 | 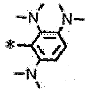 |
| **D-42** | **H** | **H** | **-NCS** | **-NCS** | 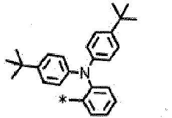 | 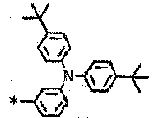 |
| **D-43** | **H** | **H** | **-NCS** | **-NCS** | 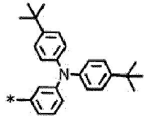 | 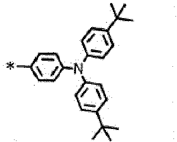 |
| **D-44** | **Na** | **H** | **-NCS** | **-NCS** | 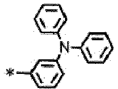 | 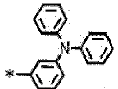 |
| **D-45** | **K** | **H** | **-NCS** | **-NCS** | 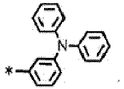 | 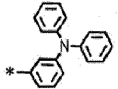 |
| **D-46** | 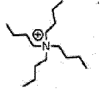 | **H** | **-NCS** | **-NCS** | 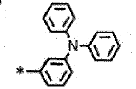 | 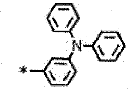 |
| **D-47** | 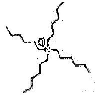 | **H** | **-NCS** | **-NCS** | 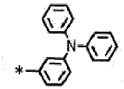 | 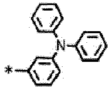 |
| **D-48** | **Na** | **H** | **-NCS** | **-NCS** | 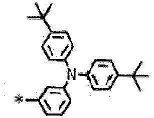 | 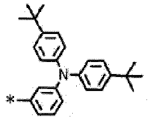 |
| **D-49** | **K** | **H** | **-NCS** | **-NCS** | 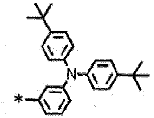 | 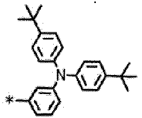 |
| **D-50** | 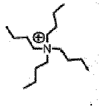 | **H** | **-NCS** | **-NCS** | 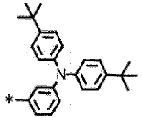 | 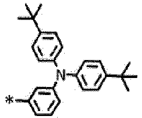 |
| **D-51** | 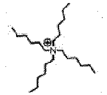 | **H** | **-NCS** | **-NCS** | 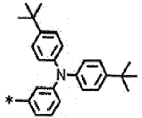 | 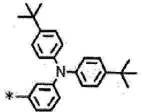 |
| **D-52** | **Na** | **H** | **-NCS** | **-NCS** | 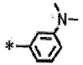 | 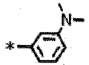 |
| **D-53** | **K** | **H** | **-NCS** | **-NCS** | 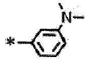 | 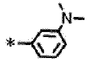 |
| **D-54** | 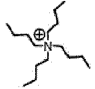 | **H** | **-NCS** | **-NCS** | 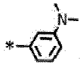 | 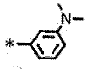 |
| **D-55** | 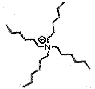 | **H** | **-NCS** | **-NCS** | 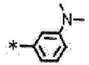 | 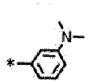 |
| **D-56** | **Na** | **Na** | **-NCS** | **-NCS** | 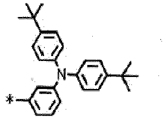 | 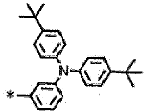 |
| **D-57** | **H** | **H** | **-NCS** | **-CN** | 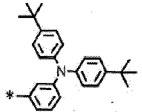 | 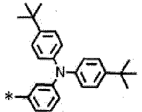 |
| **D-58** | **H** | **H** | **-NCS** | **-SCN** | 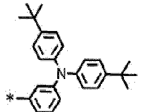 | 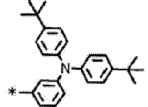 |
| **D-59** | **H** | **H** | **-SCN** | **-SCN** | 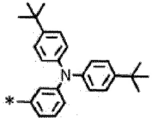 | 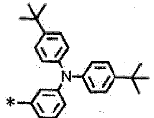 |
| **D-60** | **H** | **H** | **-NCS** | **-Cl** | 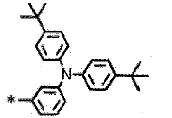 | 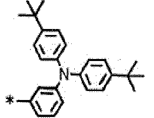 |
| **D-61** | **H** | **H** | **-SCN** | **-Cl** | 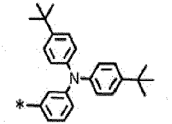 | 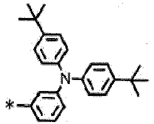 |
| **D-62** | **H** | **H** | **-Cl** | **-Cl** | 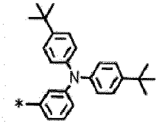 | 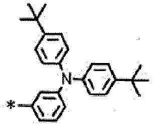 |
| **D-63** | **H** | **H** | **-NCS** | **-NCS** | 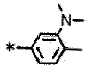 | 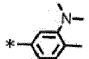 |
| **D-64** | **H** | **H** | **-NCS** | **-NCS** | 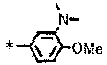 | 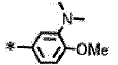 |
| **D-65** | **H** | **H** | **-NCS** | **-NCS** | 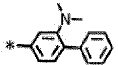 | 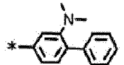 |
| **D-66** | **H** | **H** | **-NCS** | **-NCS** | 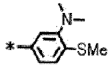 | 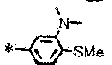 |
| **D-67** | **H** | **H** | **-NCS** | **-NCS** | 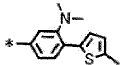 | 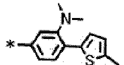 |
| **D-68** | **H** | **H** | **-NCS** | **-NCS** | 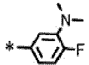 | 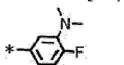 |
| **D-69** | **H** | **H** | **-NCS** | **-NCS** | 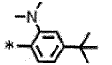 | 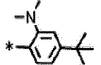 |

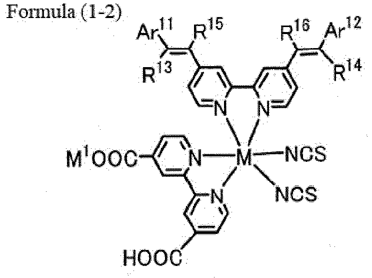

| Metal complex dye | **M¹** | **R¹³** | **R¹⁴** | **R¹⁵** | **R¹⁶** | **Ar¹¹** | **Ar¹²** |
|---|---|---|---|---|---|---|---|
| **D-70** | **H** | **Et** | **Et** | **H** | **H** | 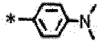 | 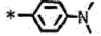 |
| **D-71** | **H** | **nBu** | **nBu** | **H** | **H** | 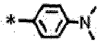 | 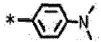 |
| **D-72** | **H** | **Ph** | **Ph** | **H** | **H** | 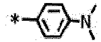 | 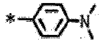 |
| **D-73** | **H** | 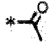 | 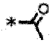 | **H** | **H** | 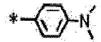 | 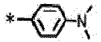 |
| **D-74** | **H** | **H** | **H** | **Et** | **Et** | 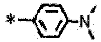 | 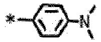 |
| **D-75** | **H** | **H** | **H** | **Ph** | **Ph** | 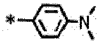 | 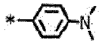 |
| **D-76** | **H** | **H** | **H** | 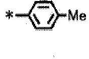 | 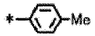 | 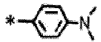 | 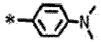 |
| **D-77** | **H** | **H** | **H** | 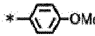 | 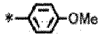 | 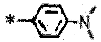 | 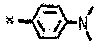 |
| **D-78** | **H** | **H** | **H** | 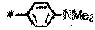 | 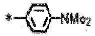 | 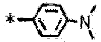 | 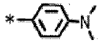 |
| **D-79** | **H** | **H** | **H** | 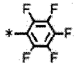 | 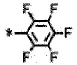 | 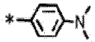 | 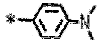 |
| **D-80** | **H** | **H** | **H** | 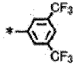 | 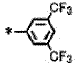 | 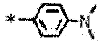 | 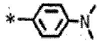 |
| **D-81** | **H** | **H** | **H** | 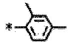 | 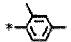 | 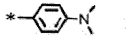 | 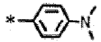 |
| **D-82** | **H** | **H** | **H** | 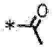 | 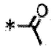 | 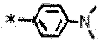 | 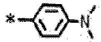 |
| **D-83** | H | **H** | **H** | 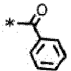 | 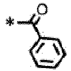 | 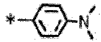 | 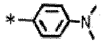 |
| **D-84** | **H** | **H** | **H** | **CH₂CF₃** | **CH₂CF₃** | 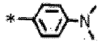 | 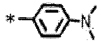 |
| **D-85** | **H** | **H** | **H** | 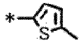 | 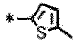 | 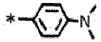 | 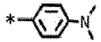 |
| **D-86** | **H** | **H** | **H** | 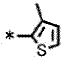 | 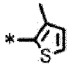 | 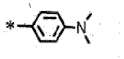 | 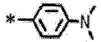 |
| **D-87** | **H** | **H** | **H** | 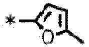 | 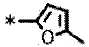 | 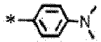 | 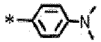 |
| **D-88** | **H** | **Et** | **Et** | **Et** | **Et** | 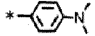 | 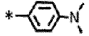 |
| **D-89** | **H** | **Et** | **Et** | **H** | **H** | 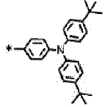 | 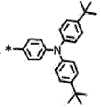 |
| **D-90** | **H** | **H** | **H** | **Et** | **Et** | 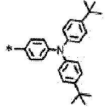 | 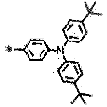 |
| **D-91** | **H** | **H** | **H** | **Ph** | **Ph** | 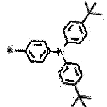 | 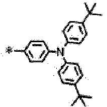 |
| **D-92** | **H** | **Me** | **Me** | **H** | **H** | 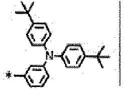 | 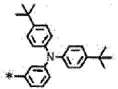 |
| **D-93** | **H** | **Et** | **Et** | **H** | **H** | 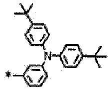 | 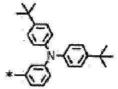 |
| **D-94** | **H** | **H** | **H** | **Me** | **Me** | 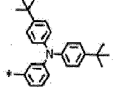 | 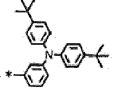 |
| **D-95** | **H** | **H** | **H** | **Et** | **Et** | 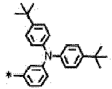 | 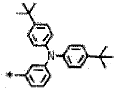 |
| **D-96** | **H** | **H** | **H** | **Ph** | **Ph** | 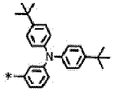 | 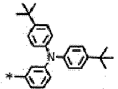 |
| **D-97** | **H** | **Me** | **Me** | **H** | **H** | 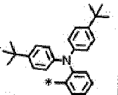 | 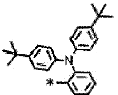 |
| **D-98** | **H** | **Et** | **Et** | **H** | **H** | 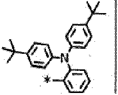 | 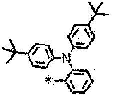 |
| **D-99** | **H** | **H** | **H** | **Me** | **Me** | 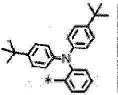 | 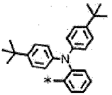 |
| **D-100** | **H** | **H** | **H** | **Et** | **Et** | 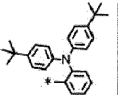 | 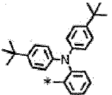 |
| **D-101** | **H** | **H** | **H** | **Ph** | **Ph** | 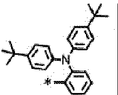 | 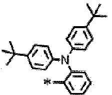 |
| **D-102** | **H** | **H** | **H** | **Et** | **Et** | 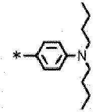 | 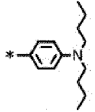 |
| **D-103** | **H** | **H** | **H** | **Et** | **Et** | 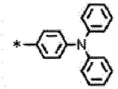 | 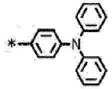 |
| **D-104** | **H** | **H** | **H** | **Et** | **Et** | 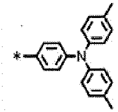 | 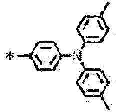 |
| **D-105** | **H** | **H** | **H** | **Et** | **Et** | 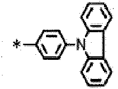 | 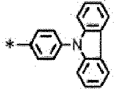 |
| **D-106** | **H** | **Et** | **H** | **Et** | **H** | 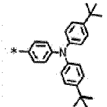 | 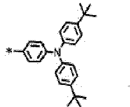 |
| **D-107** | **H** | **Et** | **H** | **H** | **Et** | 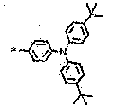 | 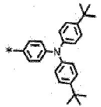 |
| **D-108** | **H** | **Et** | **nPr** | **H** | **H** | 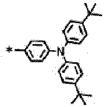 | 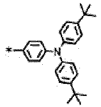 |
| **D-109** | **H** | **Et** | **Ph** | **H** | **H** | 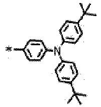 | 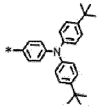 |
| **D-110** | **H** | **Me** | **Et** | **H** | **H** | 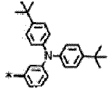 | 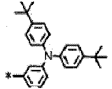 |
| **D-111** | **H** | **Me** | **H** | **Me** | **H** | 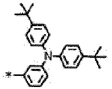 | 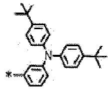 |
| **D-112** | **H** | **Et** | **Et** | **H** | **H** | 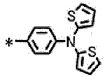 | 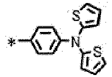 |
| **D-113** | **H** | **H** | **H** | **H** | **H** | 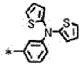 | 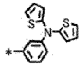 |
| **D-114** | **H** | **Et** | **Et** | **H** | **H** | 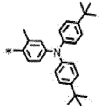 | 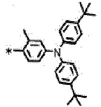 |
| **D-115** | **H** | **Et** | **Et** | **H** | **H** | 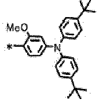 | 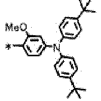 |
| **D-116** | **H** | **Et** | **Et** | **H** | **H** | 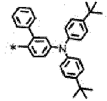 | 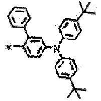 |
| **D-117** | **H** | **Et** | **Et** | **H** | **H** | 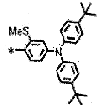 | 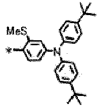 |
| **D-118** | **H** | **Et** | **Et** | **H** | **H** | 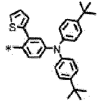 | 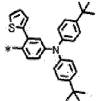 |
| **D-119** | **H** | **Et** | **Et** | **H** | **H** | 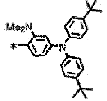 | 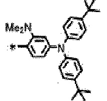 |
| **D-120** | **H** | **Et** | **Et** | **H** | **H** | 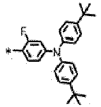 | 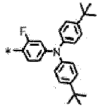 |
| **D-121** | **H** | **Et** | **Et** | **H** | **H** | 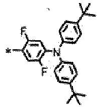 | 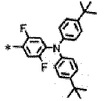 |
| **D-122** | **H** | **Et** | **Et** | **H** | **H** | 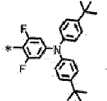 | 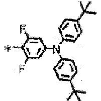 |
| **D-123** | **H** | **Et** | **Et** | **H** | **H** | 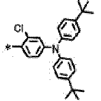 | 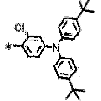 |
| **D-124** | **H** | **H** | **H** | **H** | **H** | 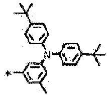 | 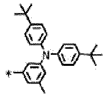 |
| **D-125** | **H** | **H** | **H** | **H** | **H** | 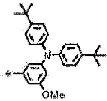 | 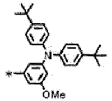 |
| **D-126** | **H** | **H** | **H** | **H** | **H** | 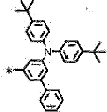 | 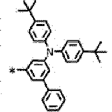 |
| **D-127** | **H** | **H** | **H** | **H** | **H** | 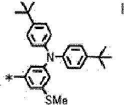 | 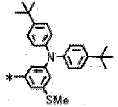 |
| **D-128** | **H** | **H** | **H** | **H** | **H** | 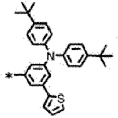 | 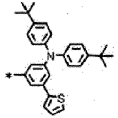 |
| **D-129** | **H** | **H** | **H** | **H** | **H** | 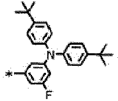 | 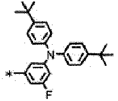 |
| **D-130** | **H** | **H** | **H** | **H** | **H** | 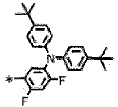 | 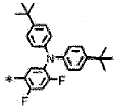 |

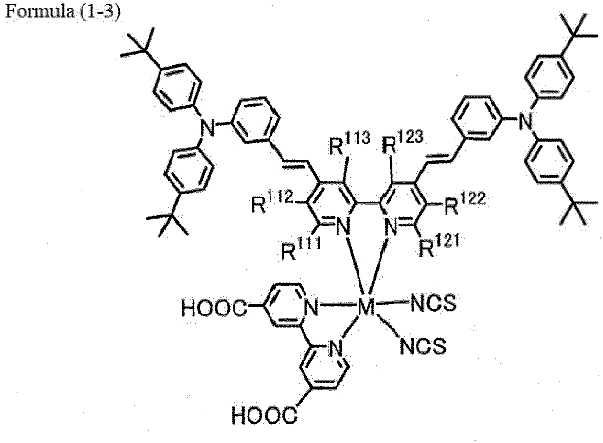

| Metal complex dye | **R¹¹¹** | **R¹¹²** | **R¹¹³** | **R¹²¹** | **R¹²²** | **R¹²³** |
|---|---|---|---|---|---|---|
| **D-131** | **H** | **Me** | **H** | **H** | **H** | **H** |
| **D-132** | **H** | **Ph** | **H** | **H** | **H** | **H** |
| **D-133** | **H** | **Me** | **H** | **H** | **Ph** | **H** |
| **D-134** | **H** | **Me** | **H** | **H** | **Me** | **H** |
| **D-135** | **H** | **OMe** | **H** | **H** | **OMe** | **H** |
| **D-136** | **H** | **Ph** | **H** | **H** | **Ph** | **H** |
| **D-137** | **H** | **SMe** | **H** | **H** | **SMe** | **H** |
| **D-138** | **H** | 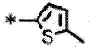 | **H** | **H** | 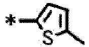 | **H** |
| **D-139** | **H** | **NMe₂** | **H** | **H** | **NMe₂** | **H** |
| **D-140** | **H** | **F** | **H** | **H** | **F** | **H** |
| **D-141** | **H** | **F** | **H** | **H** | **F** | **H** |
| **D-142** | **F** | **H** | **H** | **F** | **H** | **H** |
| **D-143** | **F** | **F** | **H** | **F** | **F** | **H** |
| **D-144** | **F** | **H** | **F** | **F** | **H** | **F** |
| **D-145** | **H** | **Cl** | **H** | **H** | **Cl** | **H** |
| **D-146** | **H** | **Br** | **H** | **H** | **Br** | **H** |
| **D-147** | **H** | **I** | **H** | **H** | **I** | **H** |

Next, preferred aspects of the main members of the photoelectric conversion element and the dye-sensitized solar cell will be described.

### <Electrically Conductive Support>

The electrically conductive support is not particularly limited as long as it has electrical conductivity and is capable of supporting a photoconductor layer 2 or the like. The electrically conductive support is a material having electrical conductivity, for example, preferably an electrically conductive support 1 formed of a metal which will be described later, or an electrically conductive support 41 having a glass or plastic substrate 44 and a transparent electrically-conductive film 43 formed on the surface of the substrate 44.

Among those, the electrically conductive support 41 having the transparent electrically-conductive film 43 of a metal oxide on the surface of the substrate 44 is more preferable. Such the electrically conductive support 41 is obtained by applying an electrically conductive metal oxide on the surface of the substrate 44 to form the transparent electrically-conductive film 43. Examples of the substrate 44 formed of plastics include the transparent polymer films described in paragraph No. 0153 of JP2001-291534A. Further, as a material which forms the substrate 44, ceramics (JP2005-135902A) or electrically conductive resins (JP2001-160425A) can be used, in addition to glass and plastics. As the metal oxide, tin oxide (TO) is preferable, and indium-tin oxide (tin-doped indium oxide; ITO) and fluorine-doped tin oxide (FTO) such as tin oxide which has been doped with tin are particularly preferable. In this case, the coating amount of the metal oxide is preferably 0.1 to 100 g, per square meter of the surface area of the substrate 44. In a case of using the electrically conductive support 41, it is preferable that light is incident from the substrate 44.

It is preferable that the electrically conductive supports 1 and 41 are substantially transparent. The expression, "substantially transparent", means that the transmittance of light (at a wavelength of 300 to 1,200 nm) is 10% or more, preferably 50% or more, and particularly preferably 80% or more.

The thickness of the electrically conductive supports 1 and 41 is not particularly limited, but is preferably 0.05 µm to 10 mm, more preferably 0.1 µm to 5 mm, and particularly preferably 0.3 µm to 4 mm.

In a case where the transparent electrically-conductive film 43 is included, the thickness of the transparent electrically-conductive film 43 is preferably 0.01 to 30 µm, more preferably 0.03 to 25 µm, and particularly preferably 0.05 to 20 µm.

It is preferable that the electrically conductive supports 1 and 41 have a metal oxide coating film including a metal oxide on the surface thereof. As the metal oxide, the metal oxide that forms the transparent electrically-conductive film 43 or the metal oxide mentioned as the metal oxide as the semiconductor fine particles which will be described later can be used, and the metal oxide mentioned as the semiconductor fine particles is preferable. The metal oxide may be a metal oxide which is the same as or different from the metal oxide that forms the transparent electrically-conductive film 43 or the metal oxide mentioned as the semi-conductive fine particles. The metal oxide coating film is usually formed on a thin film, and preferably has a thickness of 0.01 to 100 nm, for example. A method for forming the metal oxide coating film is not particularly limited, and examples thereof include the same method as the method for forming a layer formed by the semiconductor fine particles which will be described later. For example, a liquid including a metal oxide or a precursor thereof (for example, a halide and an alkoxide) can be applied and heated (calcined) to form a metal oxide coating film.

The electrically conductive supports 1 and 41 may be provided with a light management function at the surface, and may have, for example, the anti-reflection film having a high refractive index film and a low refractive index oxide film alternately laminated described in JP2003-123859A, and the light guide function described in JP2002-260746A on the surface.

### <Photoconductor Layer>

As long as the photoconductor layer has semiconductor fine particles 22 having the dye 21 supported thereon and an electrolyte, it is not particularly limited in terms of the other configurations. Preferred examples thereof include the photoconductor layer 2 and the photoconductor layer 42.

### - Semiconductor Fine Particles (Layer Formed by Semiconductor Fine Particles) -

The semiconductor fine particles 22 are preferably fine particles of chalcogenides of metals (for example, oxides, sulfides, and selenides) or of compounds having perovskite type crystal structures. Preferred examples of the chalcogenides of metals include oxides of titanium, tin, zinc, tungsten, zirconium, hafnium, strontium, indium, cerium, yttrium, lanthanum, vanadium, niobium, or tantalum; cadmium sulfide; and cadmium selenide. Preferred examples of the compounds having perovskite type crystal structures include strontium titanate and calcium titanate. Among these, titanium oxide (titania), zinc oxide, tin oxide, and tungsten oxide are particularly preferable.

Examples of the crystal structure of titania include structures of an anatase type, a brookite type, and a rutile type, and the structures of an anatase type and a brookite type are preferable. A titania nanotube, nanowire, or nanorod may be used singly or in mixture with titania fine particles.

The particle diameter of the semiconductor fine particles 22, which is expressed in terms of an average particle diameter using a diameter in a case where a projected area is converted into a circle, is preferably 0.001 to 1 µm as primary particles, and 0.01 to 100 µm as an average particle diameter of dispersions.

It is preferable that the semiconductor fine particles 22 have a large surface area so that they may adsorb a large amount of the dye 21. For example, in a state where the semiconductor fine particles 22 are coated on the electrically conductive support 1 or 41, the surface area is preferably 10 times or more, and more preferably 100 times or more, with respect to the projected area. The upper limit of this value is not particularly limited, but is usually approximately 5,000 times. In general, as the thickness of the layer (photoconductor layer) formed by the semiconductor fine particles increases, the amount of dye 21 that can be supported per unit area increases, and therefore, the light absorption efficiency increases. However, since the diffusion distance of generated electrons increases correspondingly, a loss due to charge recombination also increases.

A preferred thickness of the layer formed with the semiconductor fine particles may vary depending on the utility of the photoelectric conversion element, but typically, it is preferably 0.1 to 100 µm, more preferably 1 to 50 µm, and still more preferably 3 to 30 µm.

The layer of the semiconductor fine particles 22 can be formed by, for example, applying the semiconductor fine particles 22 onto the electrically conductive support 1 or 41, and then calcining them at a temperature of 100°C to 800°C for 10 minutes to 10 hours. Thus, the semiconductor fine particles can be adhered to each other, which is thus preferable.

Examples of the method for coating the semiconductor fine particles 22 on the electrically conductive supports 1 or 41 include a wet method, a dry method, and other methods. The coating amount of the semiconductor fine particles 22 per square meter of the surface area of the electrically conductive support 1 or 41 is preferably 0.5 to 500 g, and more preferably 5 to 100 g.

The film-forming temperature is preferably 60°C to 600°C in a case where glass is used as a material for the electrically conductive support 1 or substrate 44.

### - Light-Scattering Layer -

In the present invention, the light-scattering layer is different from the semiconductor layer in that the light-scattering layer has a function of scattering incident light.

In the dye-sensitized solar cell 20, the light-scattering layer 46 preferably contains rod-shaped or plate-shaped metal oxide particles. Examples of the metal oxide to be used in the light-scattering layer 46 include the chalcogenides (oxides) of the metals, described above as the compound which forms semiconductor fine particles. In a case of providing the light-scattering layer 46, it is preferable that the thickness of the light-scattering layer is set to 10% to 50% of the thickness of the photoconductor layer.

The light-scattering layer 46 is preferably the light-scattering layer described in JP2002-289274A, and the description in JP2002-289274A is preferably herein incorporated by reference.

### - Metal Oxide Coating Film -

In the present invention, semiconductor fine particles which form a photoconductor layer (including a case of forming the semiconductor layer 45 and the light-scattering layer 46) preferably have a metal oxide coating film on the surface thereof. As the metal oxide which forms a metal oxide coating film, the metal oxide mentioned as the semiconductor fine particles can be used, and the metal oxide may be the same as or different from the semiconductor fine particles. This metal oxide coating film is usually formed on a thin film, and preferably has a thickness of 0.1 to 100 nm, for example. In the present invention, in a case where the semiconductor fine particles have a metal oxide coating film, the metal complex dye is adsorbed on the semiconductor fine particles via the metal oxide coating film. A method for forming the metal oxide coating film is as described above.

In the present invention, in particular, it is preferable that the surfaces of the electrically conductive support and the semiconductor fine particles each have the metal oxide coating film. In this case, each of the metal oxide coating films may be formed of the same or different kinds of metal oxides.

### -Dye-

In the photoelectric conversion element 10 and the dye-sensitized solar cell 20, at least one kind of the metal complex dye represented by Formula (1) is used as a sensitizing dye. The metal complex dye represented by Formula (1) is as described above.

In the present invention, the semiconductor fine particles may have other dyes supported thereon, in addition to the metal complex dye represented by Formula (1). The dye that can be used in combination with the metal complex dye of Formula (1) is not particularly limited, but examples thereof include an Ru complex dye, a squarylium cyanine dye, an organic dye, a porphyrin dye, and a phthalocyanine dye. As the dye which can be used in combination with the other, the Ru complex dye, the squarylium cyanine dye, or the organic dye is preferable.

The overall amount of the dye to be used is preferably 0.01 to 100 millimoles, more preferably 0.1 to 50 millimoles, and particularly preferably 0.1 to 10 millimoles, per square meter of the surface area of the electrically conductive support 1 or 41. Further, the amount of the dye to be adsorbed onto the semiconductor fine particles is preferably 0.001 to 1 millimole, and more preferably 0.1 to 0.5 millimoles, with respect to 1 g of the semiconductor fine particles. By setting the amount of the dye to such a range, the sensitization effect on the semiconductor fine particles is sufficiently obtained.

In a case where the metal complex dye represented by Formula (1) is used in combination with other dyes, the ratio of the mass of the metal complex dye represented by Formula (1)/the mass of other dyes is preferably 95/5 to 10/90, more preferably 95/5 to 50/50, still more preferably 95/5 to 60/40, particularly preferably 95/5 to 65/35, and most preferably 95/5 to 70/30.

### - Electrolyte -

The electrolyte included in the photoconductor layer is as described above.

### - Co-Adsorbent -

In the present invention, it is preferable that the semiconductor fine particles support a co-adsorbent together with the metal complex dye represented by Formula (1) or with another dye to be used in combination, if necessary. As such a co-adsorbent, a co-adsorbent having at least one acidic group (preferably a carboxyl group or a salt thereof) is preferable, and examples thereof include a fatty acid and a compound having a steroid skeleton.

The fatty acid may be a saturated fatty acid or an unsaturated fatty acid, and examples thereof include a butanoic acid, a hexanoic acid, an octanoic acid, a decanoic acid, a hexadecanoic acid, a dodecanoic acid, a palmitic acid, a stearic acid, an oleic acid, a linoleic acid, and a linolenic acid.

Examples of the compound having a steroid skeleton include cholic acid, glycocholic acid, chenodeoxycholic acid, hyocholic acid, deoxycholic acid, lithocholic acid, and ursodeoxycholic acid. The compound having a steroid skeleton is preferably cholic acid, deoxycholic acid, or chenodeoxycholic acid, and more preferably deoxycholic acid or chenodeoxycholic acid.

Preferred examples of the co-adsorbent include the compounds represented by Formula (CA) described in paragraph Nos. 0125 to 0129 of JP2014-82187A, and the description in paragraph Nos. 0125 to 0129 of JP2014-82187A are preferably incorporated herein.

By making the co-adsorbent adsorbed onto the semiconductor fine particles, the co-adsorbent exhibits an effect of suppressing the inefficient association of the metal complex dye and an effect of preventing reverse electron transfer from the surface of the semiconductor fine particles to the redox system in the electrolyte. The amount of the co-adsorbent to be used is not particularly limited, and from the viewpoint of exhibiting the above effects effectively, the amount is preferably 0.1 to 200 moles, more preferably 1 to 100 moles, and particularly preferably 2 to 50 moles, with respect to 1 mole of the metal complex dye.

### - Amine Compound-

After supporting the dye onto the semiconductor fine particles, the surface of the semiconductor fine particles may be treated using an amine compound. Preferred examples of the amine compound include pyridine compounds (for example, 4-t-butylpyridine or polyvinylpyridine). These may be used as they are in a case where they are liquids, or may be used in a state where they are dissolved in an organic solvent.

### <Charge Transfer Layer>

The charge transfer layers 3 and 47 used in the photoelectric conversion element of the embodiment of the present invention are layers having a function of complementing electrons for the oxidants of the dye 21, and are provided between the light-receiving electrode 5 or 40 and the counter electrode 4 or 48.

The charge transfer layers 3 and 47 include electrolytes. Here, the expression, "the charge transfer layer includes an electrolyte", is meant to encompass both of an aspect in which the charge transfer layer consists of only electrolytes and an aspect in which the charge transfer layer consists of electrolytes and materials other than the electrolytes.

The charge transfer layers 3 and 47 may be any of a solid form, a liquid form, a gel form, or a mixture thereof.

### - Electrolyte -

Examples of the electrolyte include a liquid electrolyte having a redox couple dissolved in an organic solvent, and a so-called gel electrolyte in which a molten salt containing a redox couple and a liquid having a redox couple dissolved in an organic solvent are impregnated in a polymer matrix. Among those, the liquid electrolyte is preferable from the viewpoint of photoelectric conversion efficiency.

Examples of the redox couple include a combination of iodine and iodide (preferably an iodide salt or an iodide ionic liquid, and more preferably lithium iodide, tetrabutylammonium iodide, tetrapropylammonium iodide, and methylpropylimidazolium iodide), a combination of an alkylviologen (for example, methylviologen chloride, hexylviologen bromide, and benzylviologen tetrafluoroborate) and a reductant thereof, a combination of a polyhydroxybenzene (for example, hydroquinone and naphthohydroquinone) and an oxidant thereof, a combination of a divalent iron complex and a trivalent iron complex (for example, a combination of potassium ferricyanide and potassium ferrocyanide), and a combination of a divalent cobalt complex and a trivalent cobalt complex. Among these, a combination of iodine and an iodide, or a combination of a divalent cobalt complex and a trivalent cobalt complex is preferable, and a combination of iodine and an iodide is particularly preferable.

As the cobalt complex, the complex represented by Formula (CC) described in paragraph Nos. 0144 to 0156 of JP2014-082189A is preferable, and the description of paragraph Nos. 0144 to 0156 of JP2014-082189A is preferably incorporated in the present specification.

In a case where a combination of iodine and iodide is used as an electrolyte, it is preferable that a nitrogen-containing aromatic cation iodide salt of a 5- or 6-membered ring is additionally used.

The organic solvent which is used in a liquid electrolyte and a gel electrolyte is not particularly limited, but is preferably an aprotic polar solvent (for example, acetonitrile, propylene carbonate, ethylene carbonate, dimethylformamide, dimethylsulfoxide, sulfolane, 1,3-dimethylimidazolinone, and 3-methyloxazolidinone).

In particular, as the organic solvent which is used for a liquid electrolyte, a nitrile compound, an ether compound, an ester compound, or the like is preferable, a nitrile compound is more preferable, and acetonitrile or methoxypropionitrile is particularly preferable.

As a molten salt or a gel electrolyte, those described in paragraph No. 0205 and paragraph Nos. 0208 to 0213 of JP2014-139931A are preferable, and those described in paragraph No. 0205 and paragraph Nos. 0208 to 0213 of JP2014-139931A are preferably incorporated herein.

The electrolyte may contain aminopyridine compounds, benzimidazole compounds, aminotriazole compounds, aminothiazole compounds, imidazole compounds, aminotriazine compounds, urea compounds, amide compounds, pyrimidine compounds, and heterocycles not including nitrogen, in addition to pyridine compounds such as 4-t-butylpyridine, as an additive.

Moreover, a method of controlling the moisture content of the electrolytic solution may be employed in order to enhance the photoelectric conversion efficiency. Preferred examples of the method of controlling the moisture content include a method of controlling the concentration, and a method of adding a dehydrating agent. The moisture content (content ratio) of the electrolytic solution is preferably adjusted to 0% to 0.1% by mass.

Iodine can also be used as a clathrate compound of iodine with cyclodextrin. Furthermore, a cyclic amidine may be used, or an antioxidant, a hydrolysis inhibitor, a decomposition inhibitor, or zinc iodide may be added.

A solid-state charge transport layer such as a p-type semiconductor or a hole transport material, for example, CuI and CuNCS, may be used in place of the liquid electrolyte and the quasi-so lid-state electrolyte (gel electrolyte) as described above. Moreover, the electrolytes described in Nature, vol. 486, p. 487 (2012) and the like may also be used. For a solid-state charge transport layer, an organic hole transport material may be used. With regard to the organic hole transporting material, those described in paragraph No. 0214 of JP2014-139931A are preferably described, and those described in paragraph No. 0214 of JP2014-139931A are preferably incorporated herein.

The redox couple serves as an electron carrier, and therefore, it is preferably contained at a certain concentration. The concentration of the redox couple in total is preferably 0.01 mol/L or more, more preferably 0.1 mol/L or more, and particularly preferably 0.3 mol/L or more. In this case, the upper limit is not particularly limited, but is usually approximately 5 mol/L.

### <Counter Electrode>

It is preferable that the counter electrodes 4 and 48 work as a positive electrode in a dye-sensitized solar cell. The counter electrodes 4 and 48 usually have the same configurations as the electrically conductive support 1 or 41, but in a configuration in which strength is sufficiently maintained, a substrate 44 is not necessarily required.

Examples of a material having electrical conductivity which forms a counter electrode include, in addition to those described with respect to the electrically conductive support 41, metals such as platinum, gold, nickel, copper, silver, indium, ruthenium, palladium, rhodium, iridium, osmium, and aluminum, carbon materials, and electrically conductive polymers.

A preferred structure of the counter electrodes 4 and 48 is a structure having a high charge collecting effect. At least one of the electrically conductive support 1 or 41 and the counter electrode 4 or 48 should be substantially transparent so that light may reach the photoconductor layers 2 and 42. In the dye-sensitized solar cell of the embodiment of the present invention, the electrically conductive support 1 or 41 is preferably transparent to allow solar light to be incident from the side of the electrically conductive support 1 or 41. In this case, the counter electrodes 4 and 48 more preferably have light reflecting properties. As the counter electrodes 4 and 48 of the dye-sensitized solar cell, glass or plastic on which a metal or an electrically conductive oxide is deposited is preferable, and glass on which platinum is deposited is particularly preferable.

The film thickness of the counter electrode is not particularly limited, and is preferably 0.01 to 100 µm, more preferably 0.01 to 10 µm, and particularly preferably 0.01 to 1 µm.

### <Other Configurations>

It is preferable that a short circuit-preventing layer is formed between the electrically conductive support 1 or 41 and the photo conductor layer 2 or 42 so as to prevent reverse current due to a direct contact between the electrolyte included in the photoconductor layer 2 or 42 and the electrically conductive support 1 or 41.

Incidentally, it is preferable to use a spacer S (see Fig. 2) and/or a separator so as to prevent contact between the light-receiving electrode 5 or 40 and the counter electrode 4 or 48.

In addition, in the photoelectric conversion element or the dye-sensitized solar cell, a lateral side of the photoelectric conversion element or the dye-sensitized solar cell is preferably sealed with a polymer, an adhesive, or the like in order to prevent evaporation of components.

The dye-sensitized solar cell of the embodiment of the present invention is configured using the above-mentioned photoelectric conversion element. For example, the electrically conductive support and the counter electrode of the photoelectric conversion element are connected with an external circuit 6 to form a dye-sensitized solar cell as shown in Fig. 1. Known external circuits can also be used as the external circuit 6 without particular limitation.

The photoelectric conversion element and the dye-sensitized solar cell each have the metal complex dye represented by Formula (1) supported thereon. Thus, it can be expected that a high open-circuit voltage is exhibited and the photoelectric conversion efficiency is improved. This also applies to those in a low-illumination environment.

### [Method for Producing Photoelectric Conversion Element and Dye-Sensitized Solar Cell]

The photoelectric conversion element and the dye-sensitized solar cell of the embodiments of the present invention are each preferably produced using the dye solution of an embodiment of the present invention (also referred to as a dye composition) which contains the metal complex dye represented by Formula (1) and a solvent.

Such a dye solution is formed of the metal complex dye of the embodiment of the present invention dissolved in a solvent, and may also include other components, if necessary.

Examples of the solvent to be used include the solvents described in JP2001-291534A, but are not particularly limited thereto. In the present invention, an organic solvent is preferable, and an alcohol solvent, an amide solvent, a nitrile solvent, a ketone solvent, a hydrocarbon solvent, and a mixed solvent of two or more kinds of these solvents are more preferable. As the mixed solvent, a mixed solvent of an alcohol solvent and a solvent selected from an amide solvent, a nitrile solvent, a ketone solvent, and a hydrocarbon solvent is preferable; a mixed solvent of an alcohol solvent and an amide solvent, a mixed solvent of an alcohol solvent and a hydrocarbon solvent, and a mixed solvent of an alcohol solvent and a nitrile solvent are more preferable; and a mixed solvent of an alcohol solvent and an amide solvent, and a mixed solvent of an alcohol solvent and a nitrile solvent are particularly preferable. Specifically, a mixed solvent of at least one of methanol, ethanol, propanol, or t-butanol, and at least one of dimethylformamide or dimethylacetamide, and a mixed solvent of at least one of methanol, ethanol, propanol, or t-butanol, and acetonitrile are preferable.

The dye solution preferably contains a co-adsorbent, and as the co-adsorbent, the afore-mentioned co-adsorbent is preferable.

Here, the dye solution of the embodiment of the present invention is preferably one in which the concentration of the metal complex dye or the co-adsorbent has been adjusted so that the dye solution can be used as it is during production of the photoelectric conversion element or the dye-sensitized solar cell. In the present invention, the dye solution of the embodiment of the present invention preferably contains 0.001% to 0.1% by mass of the metal complex dye of the embodiment of the present invention. The amount of the co-adsorbent to be used is as described above.

It is preferable that the dye solution has a small moisture content in terms of dye adsorption. For example, the moisture content is preferably adjusted to 0% to 0.1% by mass at least during a use. The moisture content can be adjusted by an ordinary method at least during a use.

In the present invention, it is preferable to manufacture a photoconductor layer by making the metal complex dye represented by Formula (1) or a dye including the same supported on the surface of the semiconductor fine particles, using the dye solution. That is, the photoconductor layer is preferably formed by applying the dye solution onto the semiconductor fine particles provided on the electrically conductive support (including a dip method), followed by drying or curing.

By further providing a charge transfer layer, a counter electrode, or the like for a light-receiving electrode comprising the photoconductor layer as manufactured above by an ordinary method, the photoelectric conversion element of the embodiment of the present invention can be obtained.

In addition, a dye-sensitized solar cell can be produced by connecting an external circuit 6 with the electrically conductive support 1 and the counter electrode 4 of the photoelectric conversion element thus manufactured.

### Examples

Hereinafter, the present invention will be described in more detail, based on Examples, but is not limited thereto.

### Example 1 (Synthesis of Metal Complex Dye)

Hereinafter, the compounds of the metal complex dye of the embodiment of the present invention and methods for synthesizing the same will be described in detail, but the starting materials, the dye intermediates, and the synthesis routes are not limited thereto.

In the present invention, a room temperature means 25°C.

### <Synthesis of Metal Complex Dye D-4>

According to a method of the following scheme, a metal complex dye D-4 was synthesized.

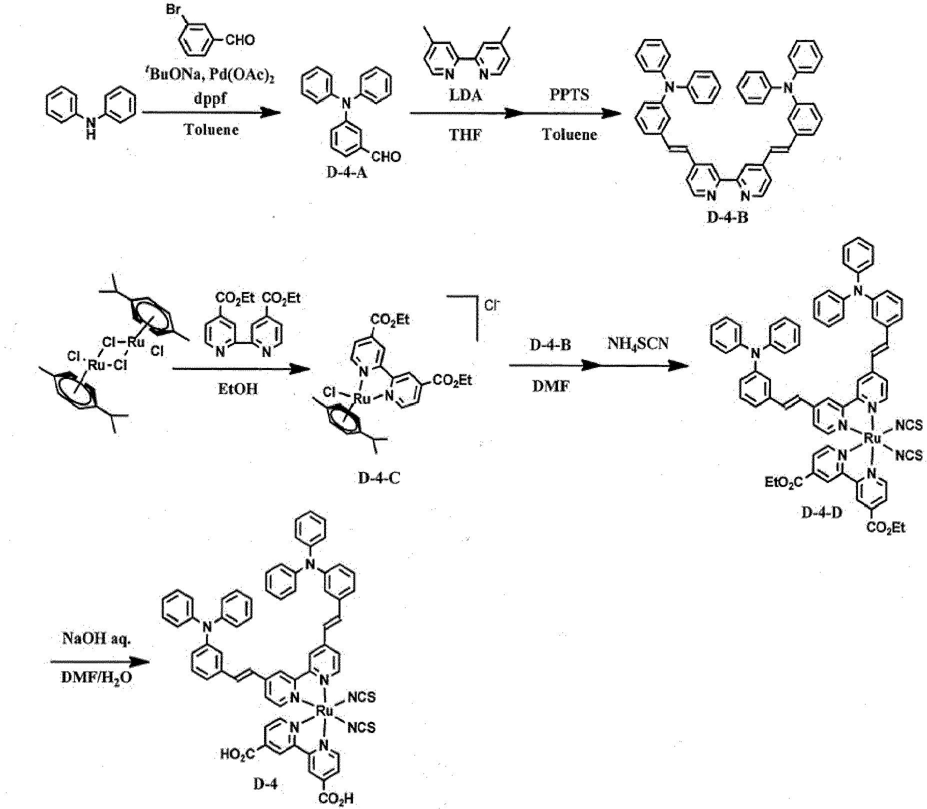

### (i) Synthesis of Compound D-4-A

16.9 g of diphenyl amine was dissolved in 200 mL of toluene and stirred at room temperature in a 1-L 3-necked flask, 10.6 g of t-butoxysodium was added thereto, and the mixture was deaerated. In a nitrogen gas atmosphere, 1.39 g of 1,1'-bis(diphenylphosphino)ferrocene (dppf) and 0.56 g of palladium (II) acetate were added to the obtained solution, then 19.4 g of 3-bromobenzaldehyde was further added thereto, and the mixture was stirred at 100°C for 1 hour. The obtained mixture was cooled to room temperature, then 150 mL of water, 75 mL of ethyl acetate, and 10 mL of methanol were added thereto, and the mixture was extracted and subjected to liquid separation. Saturated physiological saline was further added to the organic layer and the mixture was subjected to liquid separation. The obtained organic layer was filtered through Celite and the liquid was concentrated. A crude product thus obtained was purified by silica gel column chromatography to obtain 17.5 g of a compound D-4-A.

### (ii) Synthesis of Compound D-4-B

In a nitrogen gas atmosphere, 3.68 g of 4,4'-dimethyl-2,2'-bipyridine and 100 mL of dehydrated tetrahydrofuran (THF) were put into a 500-mL 3-necked flask, and the mixture was cooled to -15°C. 31 mL of a 1.5 M (moles/L) THF solution of lithium diisopropylamide (LDA) was added dropwise thereto and the mixture was stirred for 1 hour. Thereafter, a solution in which 12.0 g of the compound D-4-A was dissolved in 30 mL of THF was added dropwise to the obtained mixture, and the mixture was stirred at 0°C for 1 hour and further stirred at room temperature for 2 hours. 60 mL of a saturated aqueous ammonium chloride solution was added to the obtained mixture and 100 mL of ethyl acetate was then added thereto. The mixture was extracted and subjected to liquid separation and the organic layer was concentrated. The obtained oily residue was dissolved to in 100 mL of toluene, 10.5 g of a pyridinium paratoluenesulfonic acid anion (PPTS) was added thereto, and the mixture was heated and refluxed for 2 hours in a nitrogen atmosphere. 15 mL of triethylamine and 150 mL of methanol were added to the obtained reaction product and the precipitated solid was collected by filtration to obtain 7.0 g of a compound D-4-B.

### (iii) Synthesis of Compound D-4-C

15.3 g of a dichloro(p-cymene)ruthenium dimer and 15.0 g of 4,4'-bis(ethoxycarbonyl)-2,2'-bipyridine were heated and stirred in ethanol (EtOH) for 2 hours and then concentrated to obtain 30.3 g of a compound D-4-C.

### (iv) Synthesis of Compound D-4-D

695 mg of the compound D-4-B and 607 mg of the compound D-4-C were mixed with 20 mL of dimethylformamide (DMF), and the mixture was heated and stirred at 150°C for 7 hours. Thereafter, 1.5 g of ammonium thiocyanate was added to the obtained mixture and the mixture was further at 130°C for 5 hours. 30 mL of water was added to the obtained reaction product and the precipitated solid was collected by filtration. This crude product was purified by silica gel column chromatography to obtain 897 mg of a compound D-4-D.

### (v) Synthesis of Exemplified Dye D-4

20 mL of DMF, 2 mL of water, and 0.8 mL of a 3 N aqueous sodium hydroxide solution were added to 600 mg of the compound D-4-D, and the mixture was stirred at 30°C for 1 hour. Thereafter, a 1 N aqueous trifluoromethanesulfonic acid solution was added dropwise to the obtained mixture to adjust the pH to 3.0. The obtained reaction product was filtered to obtain 580 mg of a metal complex dye D-4.

### <Synthesis of Metal Complex Dyes D-6, 11, 21, 22, 26, 37, 43, 44, 46, 89, 90, 92, and 94>

By the same synthesis method as for the metal complex dye D-4, each of the metal complex dyes was synthesized.

### <Identification of Metal Complex Dye>

The structure of each of the metal complex dyes synthesized as described above was confirmed by mass spectroscopy (MS) measurement. The results thereof are shown in Table 1.

**[Table 1]**

| Metal complex dye | **MS(ESI⁺)** |
|---|---|
| **D-4** | **MS(ESI⁺)m/z:11157 ([M+H]⁺)** |
| **D-6** | **MS(ESI⁺)m/z:1381 ([M+H]⁺)** |
| **D-11** | **MS(ESI⁺)m/z:1381 ([M+H]⁺)** |
| **D-21** | **MS(ESI⁺)m/z:1257 ([M+H]⁺)** |
| **D-22** | **MS(ESI⁺)m/z:1257 ([M+H]⁺)** |
| **D-26** | **MS(ESI⁺)m/z:1153 ([M+H]⁺)** |
| **D-37** | **MS(ESI⁺)m/z:1157 (M+H]⁺)** |
| **D-43** | **MS(ESI⁺)m/z:1381 ([M+H]⁺)** |
| **D-44** | **MS(ESI⁺)m/z:1157 ([M+H]⁺)** |
| **D-46** | **MS(ESI⁺)m/z:1157 ([M+H]⁺)** |
| **D-89** | **MS(ESI⁺)m/z:1437 ([M+H]⁺)** |
| **D-90** | **MS(ESI⁺)m/z:1437 ([M+H]⁺)** |
| **D-92** | **MS(ESI⁺)m/z:1409 ([M+H]⁺)** |
| **D-94** | **MS(ESI⁺)m/z:1409 ([M+H]⁺)** |

### Example 2 (Production of Dye-Sensitized Solar Cell)

Using each of the metal complex dyes synthesized in Example 1 or the following comparative compounds (C1) to (C6), a dye-sensitized solar cell 20 (in a dimension of 5 mm × 5 mm) shown in Fig. 2 was produced and its performance was evaluated according to the procedure shown below. The results are shown in Table 2.

### (Manufacture of Light-Receiving Electrode Precursor)

An electrically conductive support 41 was prepared, in which a fluorine-doped SnO₂ electrically-conductive film (transparent electrically-conductive film 43, film thickness of 500 nm) was formed on a glass substrate (substrate 44, thickness of 4 mm). Further, a glass substrate having an SnO₂ electrically-conductive film formed thereon was immersed in a 40 mM aqueous titanium tetrachloride solution for 30 minutes, washed with ultrapure water and ethanol, and then calcined at 450°C to form a thin film layer of the titanium oxide (metal oxide coating film, not shown in Fig. 2) on the SnO₂ electrically-conductive film. A titania paste "18NR-T" (manufactured by DyeSol) was screen-printed on the thin film layer and dried at 120°C. Then, the titania paste "18NR-T" was screen-printed again and dried at 120°C for 1 hour. Thereafter, the dried titania paste was calcined at 500°C to form a semiconductor layer 45 (film thickness; 10 µm). Further, a titania paste "18NR-AO" (manufactured by DyeSol) was screen-printed on the semiconductor layer 45 and dried at 120°C for 1 hour. Thereafter, the dried titania paste was calcined at 500°C to form a light-scattering layer 46 (film thickness: 5 µm) on the semiconductor layer 45. Thus, a photoconductor layer 42 (an area of the light-receiving surface; 5 mm×5 mm and a film thickness; 15 µm) was formed on the SnO₂ electrically-conductive film. Subsequently, the glass substrate with an SnO₂ electrically-conductive film having a photoconductor layer formed thereon was immersed in a 20 mM aqueous titanium tetrachloride solution and washed with ultrapure water and ethanol to form a titanium oxide layer (metal oxide coating film, not shown in Fig. 2) on the surface of the photoconductor layer. By the procedure above, a light-receiving electrode precursor having no metal complex dye supported thereon was manufactured.

### (Dye Adsorbing Method)

Next, each of the metal complex dyes synthesized in Example 1 was supported onto the photoconductor layer 42 having no metal complex dye supported thereon in the following manner. First, each of the metal complex dyes was mixed in a mixed solvent of t-butanol and acetonitrile at 1:1 (volume ratio) such that the concentration became 2×10⁻⁴mol/L. Further, 10 mol of chenodeoxycholic acid as a co-adsorbent was added to 1 mol of the metal complex dye to prepare each of dye solutions. Next, the light-receiving electrode precursor was immersed in each of the dye solutions at 25°C for 5 hours and dried after pulling out from the dye solution, thereby manufacturing each of light-receiving electrodes 40 having each of the metal complex dyes supported on the light-receiving electrode precursor.

### (Assembly of Dye-Sensitized Solar Cell)

A platinum electrode (thickness of a Pt thin film; 100 nm) having the same shape and size as those of the electrically conductive support 41 was manufactured as the counter electrode 48. Further, as electrolytes, 0.1 M (mol/L) of iodine, 0.1 M of lithium iodide, 0.005 M of 4-t-butylpyridine, and 0.6 M of 1,2-dimethyl-3-propylimidazolium iodide were dissolved in acetonitrile to prepare a liquid electrolyte as an electrolytic solution. In addition, Spacer S (trade name: "SURLYN") manufactured by DuPont, which has a shape matching to the size of the photoconductor layer 42, was prepared.

Each of the light-receiving electrodes 40 manufactured as above and the counter electrode 48 were arranged to face each other through the Spacer S and thermally compressed, and then the liquid electrolyte was filled from the inlet for the electrolytic solution between the photoconductor layer 42 and the counter electrode 48, thereby forming a charge transfer layer 47. The outer periphery and the inlet for the electrolytic solution of the cell thus manufactured were sealed and cured using RESIN XNR-5516 manufactured by Nagase Chemtex Corporation to produce of dye-sensitized solar cells (Sample Nos. 1 to 14).

Comparative dye-sensitized solar cells (Sample Nos. c1 to c6) were produced in the same manner as for the production of the dye-sensitized solar cell, except that each of the following comparative metal complex dyes (C1) to (C6) was used instead of the metal complex dye synthesized in Example 1 in the production of the dye-sensitized solar cell.

The metal complex dye (C1) is the metal complex dye D-3 described in JP2001-291534A.

The metal complex dye (C2) is a dye referred to as N719. With regard to this dye, TBA represents tetrabutylammonium.

The metal complex dye (C3) is the compound "Ru-TPA-EO-NCS" described in J. Mater. Chem., 2009, 19, p. 5364-5376.

The metal complex dye (C4) is the metal complex dye D-1-7a described in JP2013-072079A.

The metal complex dye (C5) is the compound described as a compound No. 27 in JP2008-021496A.

The metal complex dye (C6) is the metal complex dye D-54 described in JP2001-291534A.

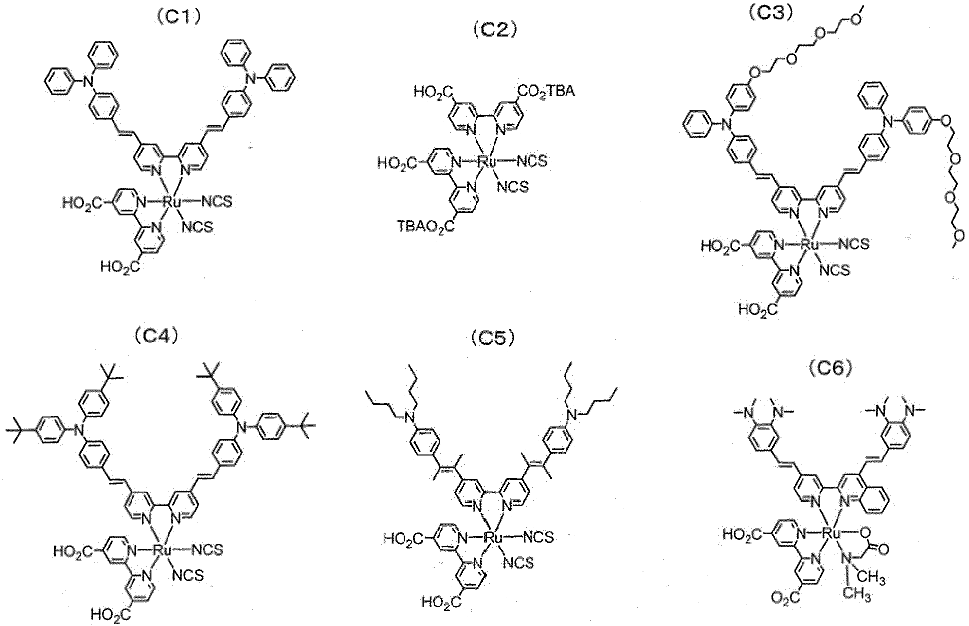

### <Evaluation of Open-Circuit Voltage>

A test on cell characteristics was performed using each of the dye-sensitized solar cells produced. The test on cell characteristics was performed by irradiating pseudo-sunlight at 1,000 W/m² from a xenon lamp, which had passed through AMI.5 filter G, onto each of photoelectric conversion modules using a solar simulator "PEC-L15" (manufactured by Peccell Technologies, Inc.). By measuring the current-voltage characteristics of each of the photoelectric conversion modules irradiated with pseudo-sunlight using a source meter "Keithley 2401" (manufactured by Tektronix, Inc.), the open-circuit voltage was measured.

A relative value (open-circuit voltage V_{OC}/V_{OC}^{C2}) of the open-circuit voltage V_{OC} measured for each of the dye-sensitized solar cells with the respective Sample Nos. with respect to the open-circuit voltage V_{OC}^{C2} of the comparative dye-sensitized solar cells (Sample No. c2) was calculated and evaluated according to the following standards (evaluation ranks). The relative value thus determined was classified to one of the following evaluation standards.

In the present test, for the evaluation of the open-circuit voltage, the evaluation ranks A to C are at acceptable levels, and the evaluation ranks A and B are at preferable levels.
A: More than 1.10
B: More than 1.09 and 1.10 or less
C: More than 1.07 and 1.09 or less
D: More than 1.05 and 1.07 or less
E: More than 1.00 and 1.05 or less
F: 1.00 or less

In the battery characteristic test, any of the respective dye-sensitized solar cell of Sample Nos. 1 to 14 showed a photoelectric conversion efficiency which sufficiently functions as a dye-sensitized photoelectrochemical cell.

**[Table 2]**

| Sample No. | Metal complex dye | Open-circuit voltage | Note |
|---|---|---|---|
| **1** | **D-4** | **A** | The present invention |
| **2** | **D-6** | **A** | The present invention |
| **3** | **D-11** | **A** | The present invention |
| **4** | **D-21** | **A** | The present invention |
| **5** | **D-22** | **A** | The present invention |
| **6** | **D-26** | **A** | The present invention |
| **7** | **D-37** | **A** | The present invention |
| **8** | **D-43** | **B** | The present invention |
| **9** | **D-44** | **A** | The present invention |
| **10** | **D-46** | **A** | The present invention |
| **11** | **D-89** | **C** | The present invention |
| **12** | **D-90** | **C** | The present invention |
| **13** | **D-92** | **A** | The present invention |
| **14** | **D-94** | **A** | The present invention |
| **c1** | **(C1)** | **D** | Comparative Example |
| **c2** | **(C2)** | Standard | Comparative Example |
| **c3** | **(C3)** | **F** | Comparative Example |
| **c4** | **(C4)** | **D** | Comparative Example |
| **c5** | **(C5)** | **D** | Comparative Example |
| **c6** | **(C6)** | **D** | Comparative Example |

From the results of Table 2, the following findings could be obtained.

Any of the comparative photoelectric conversion elements and dye-sensitized solar cells (Sample Nos. c1 to c6) did not exhibit a sufficient open-circuit voltage.

Specifically, the metal complex dyes (C1), and (C4) to (C6) in which all of R¹³ to R¹⁶ are each a hydrogen atom or methyl, and all of Ar¹¹ and Ar¹² have the group represented by Formula (2-1) in Formula (1) had an increased open-circuit voltage even though they are metal complex dyes having a ligand having a diphenylaminostyryl skeleton, but their increasing extents were small and insufficient, as compared with the metal complex dye (C2). Further, the metal complex dye (C3) in which the amino group of the diphenylaminostyryl skeleton has an ethyleneoxy group showed a decrease in the open-circuit voltage.

In contrast, it was found that any of the dye-sensitized solar cells (Sample Nos. 1 to 14) using the metal complex dye represented by Formula (1) exhibited an effect of increasing the open-circuit voltage with respect to the metal complex dye (C2).

In a case where one of R¹³ to R¹⁶ in Formula (1) is a substituent, a sufficient open-circuit voltage was exhibited even with both of Ar¹¹ and Ar¹² being each the group represented by Formula (2-1). Further, in a case where at least one of Ar¹¹ or Ar¹² in Formula (1) is the group represented by Formula (2-2), and particularly, in a case where all of Ar¹¹ and Ar¹² are each the group represented by Formula (2-2), an effect of increasing the open-circuit voltage was significant and a high open-circuit voltage was exhibited even with all of R¹³ to R¹⁶ being each a hydrogen atom.

Although the present invention has been described with reference to their embodiments, it is not intended that the present invention is not limited by any of the details of the description unless otherwise specified, but should rather be construed broadly within the spirit and scope of the present invention as set out in the accompanying claims.

The present application claims the priority based on Japanese Patent Application No. 2016-190623 filed on September 29, 2016, the contents of which are incorporated by reference into a part described herein.

### Explanation of References

1, 41: electrically conductive supports
2,42: photoconductor layers (oxide semiconductor electrodes)
21: dye
22: semiconductor fine particles
3,47: charge transfer layer
4,48: counter electrodes
5,40: light-receiving electrodes
6: external circuit
10: photoelectric conversion element
100: system in which photoelectric conversion element is applied to cell uses
M: operating means (for example, electric motor)
20: dye-sensitized solar cell
43: transparent electrically-conductive film
44: substrate
45: semiconductor layer
46: light-scattering layer
S: spacer

## Claims

1. A photoelectric conversion element comprising:
an electrically conductive support;
a photoconductor layer including an electrolyte;
a charge transfer layer including an electrolyte; and
a counter electrode,
wherein the photoconductor layer has semiconductor fine particles having a metal complex dye represented by Formula (1) supported thereon, 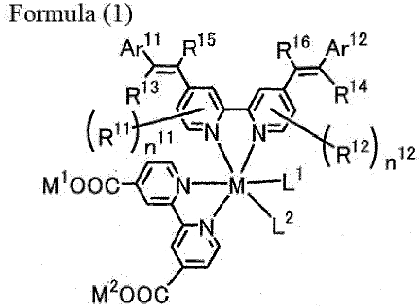
in the formula, M represents a metal ion,
R¹¹ and R¹² each independently represent an alkyl group, an alkoxy group, an aryl group, an alkylthio group, a heteroaryl group, an amino group, or a halogen atom, and n¹¹ and n¹² each independently represent an integer of 0 to 3,
R¹³ to R¹⁶ each independently represent a hydrogen atom, an alkyl group, an acyl group, an aryl group, or a heteroaryl group,
Ar¹¹ and Ar¹² each independently represent a group represented by any one of Formula (2-1) or Formula (2-2), provided that in a case where all of R¹³ to R¹⁶ are each a hydrogen atom or methyl, at least one of Ar¹¹ or Ar¹² represents a group represented by Formula (2-2),
M¹ and M² each independently represent any one of a proton, a metal cation, or a non-metal cation, and
L¹ and L² each independently represent a monodentate ligand, 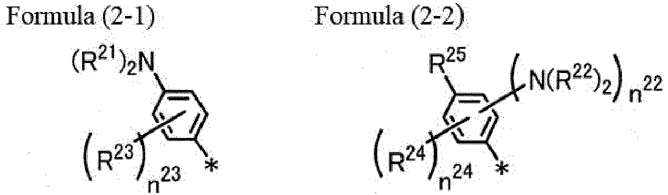
in the formulae, R²¹ and R²² each independently represent an alkyl group, an aryl group, or a heteroaryl group,
R²³ represents an alkyl group, an alkoxy group, an aryl group, an alkylthio group, a heteroaryl group, an amino group, or a halogen atom,
R²⁴ represents an alkyl group, an alkoxy group, an aryl group, an alkylthio group, a heteroaryl group, or a halogen atom,
R²⁵ represents a hydrogen atom, an alkyl group, an alkoxy group, an aryl group, an alkylthio group, a heteroaryl group, or a halogen atom,
n²² is an integer of 1 to 4, n²³ is an integer of 0 to 4, n²⁴ is an integer of 0 to 3, and a sum of n²² and n²⁴ is an integer of 1 to 4, and
* represents a bonding moiety to a carbon atom to which R¹³ or R¹⁴ is bonded.

2. The photoelectric conversion element according to claim 1,
wherein at least one of Ar¹¹ or Ar¹² represents the group represented by Formula (2-2).

3. The photoelectric conversion element according to claim 1 or 2,
wherein both of Ar¹¹ and Ar¹² each represent the group represented by Formula (2-2).

4. The photoelectric conversion element according to any one of claims 1 to 3,
wherein R²¹ and R²² each independently represent an alkyl group or an aryl group.

5. The photoelectric conversion element according to any one of claims 1 to 4,
wherein R²¹ and R²² each independently represent a phenyl group.

6. The photoelectric conversion element according to any one of claims 1 to 5,
wherein R²¹ and R²² have at least one selected from the group consisting of an alkyl group, an alkoxy group, an aryl group, an alkylthio group, a heteroaryl group, an amino group, and a halogen atom as a substituent.

7. The photoelectric conversion element according to any one of claims 1 to 6,
wherein R²¹ and R²² are each independently represented by any one of Formula (R2-1), ..., or Formula (R2-5), 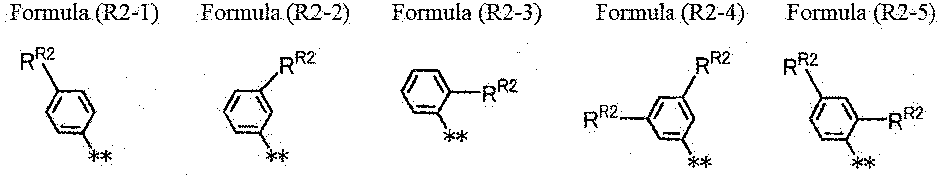 in the formulae, R^{R2} represents an alkyl group, an alkoxy group, an aryl group, an alkylthio group, a heteroaryl group, an amino group, or a halogen atom, and ** represents a bonding moiety to N in Formula (2-1) or Formula (2-2).

8. The photoelectric conversion element according to any one of claims 1 to 7,
wherein all of R¹³ to R¹⁶ are each a hydrogen atom.

9. The photoelectric conversion element according to any one of claims 1 to 7,
wherein at least one of R¹³, ..., or R¹⁶ represents an alkyl group, an acyl group, an aryl group, or a heteroaryl group.

10. The photoelectric conversion element according to claim 9,
wherein at least one of a set of R¹³ and R¹⁴ or a set of R¹⁵ and R¹⁶ represents an alkyl group, an acyl group, an aryl group, or a heteroaryl group.

11. A dye-sensitized solar cell comprising the photoelectric conversion element according to any one of claims 1 to 10.

12. A metal complex dye represented by Formula (1), 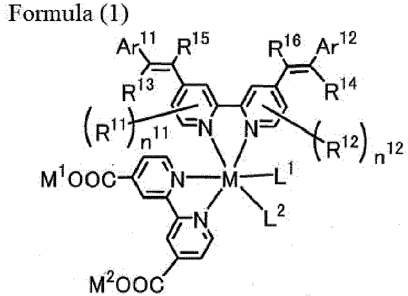
in the formula, M represents a metal ion,
R¹¹ and R¹² each independently represent an alkyl group, an alkoxy group, an aryl group, an alkylthio group, a heteroaryl group, an amino group, or a halogen atom, and n¹¹ and n¹² each independently represent an integer of 0 to 3,
R¹³ to R¹⁶ each independently represent a hydrogen atom, an alkyl group, an acyl group, an aryl group, or a heteroaryl group,
Ar¹¹ and Ar¹² each independently represent a group represented by any one of Formula (2-1) or Formula (2-2), provided that in a case where all of R¹³ to R¹⁶ are each a hydrogen atom or methyl, at least one of Ar¹¹ or Ar¹² represents a group represented by Formula (2-2),
M¹ and M² each independently represent any one of a proton, a metal cation, or a non-metal cation, and
L¹ and L² each independently represent a monodentate ligand, 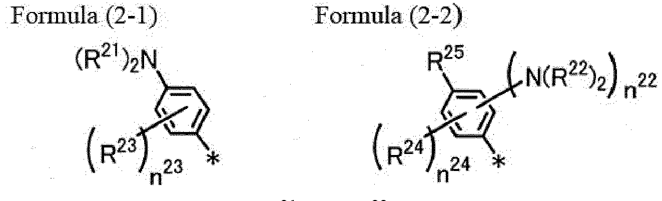
in the formulae, R²¹ and R²² each independently represent an alkyl group, an aryl group, or a heteroaryl group,
R²³ represents an alkyl group, an alkoxy group, an aryl group, an alkylthio group, a heteroaryl group, an amino group, or a halogen atom,
R²⁴ represents an alkyl group, an alkoxy group, an aryl group, an alkylthio group, a heteroaryl group, or a halogen atom,
R²⁵ represents a hydrogen atom, an alkyl group, an alkoxy group, an aryl group, an alkylthio group, a heteroaryl group, or a halogen atom,
n²² is an integer of 1 to 4, n²³ is an integer of 0 to 4, n²⁴ is an integer of 0 to 3, and a sum of n²² and n²⁴ is an integer of 1 to 4, and
* represents a bonding moiety to a carbon atom to which R¹³ or R¹⁴ is bonded.

13. A dye solution comprising:
the metal complex dye according to claim 12; and
a solvent.

14. An oxide semiconductor electrode comprising the metal complex dye according to claim 12.
